(19) Europäisches Patentamt
European Patent Office
Office européen des brevets

(11) Veröffentlichungsnummer: **0 112 802**
**B1**

(12) # EUROPÄISCHE PATENTSCHRIFT

(45) Veröffentlichungstag der Patentschrift: **07.01.88**

(51) Int. Cl.⁴: **G 03 C 7/26** // C07D211/46, C07D493/10

(21) Anmeldenummer: **83810583.1**

(22) Anmeldetag: **12.12.83**

(54) **Farbphotographisches Aufzeichnungsmaterial.**

(30) Priorität: **16.12.82 CH 7315/82**

(43) Veröffentlichungstag der Anmeldung: **04.07.84 Patentblatt 84/27**

(45) Bekanntmachung des Hinweises auf die Patenterteilung: **07.01.88 Patentblatt 88/1**

(84) Benannte Vertragsstaaten: **BE DE FR GB IT**

(56) Entgegenhaltungen: **EP-A-0 011 051**

(73) Patentinhaber: **CIBA-GEIGY AG, Klybeckstrasse 141, CH-4002 Basel (CH)**

(72) Erfinder: **Leppard, David G., Route de Bourguillon 6, CH-1723 Marly (CH)**
Erfinder: **Rody, Jean, Dr., Rütiring 82, CH-4125 Riehen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Anmeldung betrifft ein farbphotographisches Aufzeichnungsmaterial, das in mindestens einer lichtempfindlichen Silberhalogenidemulsionsschicht und/oder mindestens einer der üblichen Hilfsschichten als Stabilisator eine spezifische Polyalkylpiperidinverbindung enthält.

Polyalkylpiperidine sind als sterisch gehinderte Amine allgemein als Lichtschutzmittel für organische Materialien, insbesondere für Polymere, bekannt. Es wurde auch bereits in der DE-OS 2 126 954 vorgeschlagen, solche Polyalkylpiperidine als Mittel gegen das Ausbleichen von Farbphotrographien zu verwenden. Es wurde weiterhin in der EP-A 11051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxylbenzylmalonsäuren.

Es wurde nun gefunden, dass Polyalkylpiperidinverbindungen, die über eine Carboxyalkyliden- oder Carbaminoalkylidengruppe verknüpftes sterisch gehindertes Phenol enthalten, eine überraschend bessere stabilisierende Wirkung entfalten.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Stabilisator enthält, dadurch gekennzeichnet, dass die Polyalkylpiperidinverbindung der Formel I

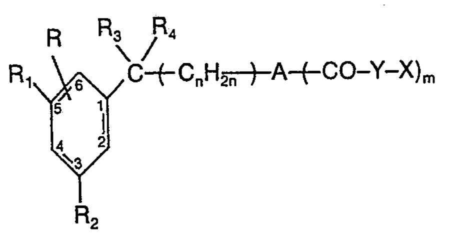

(I)

entspricht, worin

R eine OH-Gruppe ist, die sich in 2-, 4- oder 6-Stellung befindet,

$R_1$ Wasserstoff, $C_1$–$C_4$ Alkyl, eine Gruppe der Formel II

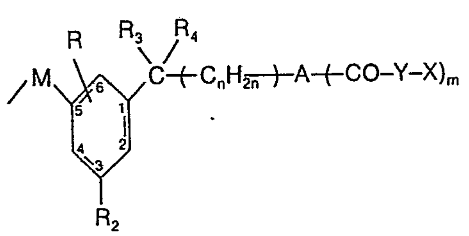

(II)

oder eine Gruppe der Formel III

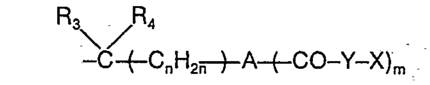

(III)

ist,

$R_2$ $C_1$–$C_4$ Alkyl, eine Gruppe der Formel III oder eine Gruppe der Formel –CO–Y–X bedeutet,

$R_3$ und $R_4$ unabhängig voneinander $C_1$–$C_8$ Alkyl sind und $R_4$ zusätzlich zusammen mit der Gruppe $-(-C_nH_{2n}-)-$ einen $C_5$–$C_{12}$ Cycloalkylrest bilden kann,

n die Zahlen 1 bis 20,

m 1 oder 2, wobei jedoch m nicht 2 ist, wenn $R_1$ für Wasserstoff oder $C_1$–$C_4$ Alkyl steht,

A eine direkte C–C-Bindung, wenn m = 1 ist, oder ein Rest –CH–, wenn m = 2 ist,

Y –O– oder $-N(R_5)-$, worin $R_5$ Wasserstoff, $C_1$–$C_{18}$ Alkyl, $C_3$–$C_{12}$ Alkenyl, $C_3$–$C_{12}$ Cycloalkyl, Phenyl, $C_7$–$C_{14}$ Aralkyl, $C_7$–$C_{14}$ Alkaryl, $C_2$–$C_{11}$ Alkoxyalkyl oder eine Gruppe der Formel IV

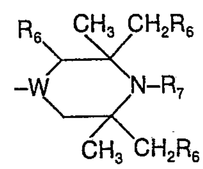

(IV)

ist, bedeutet,

M eine direkte Bindung, –O–, –S–, –S–S–, –SO–, $-SO_2-$ oder eine Gruppe $-CH_2OCH_2-$, $-CH_2SCH_2-$, $-CH(R_8)-$ oder $-N(R_9)-$ ist, worin $R_8$ Wasserstoff, $C_1$–$C_{12}$ Alkyl oder durch 1–3 Schwefelatome unterbrochenes $C_3$–$C_8$ Alkyl und $R_9$ Wasserstoff, $C_1$–$C_{18}$ Alkyl, unsubstituiertes oder durch $C_1$–$C_4$ Alkyl substituiertes Phenyl oder Benzyl bedeuten

X eine Gruppe der Formel

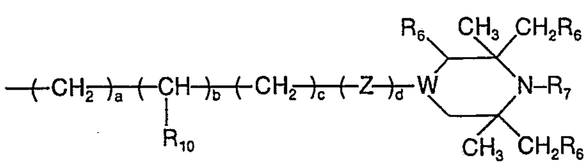

oder der Formel

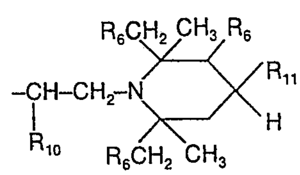

ist,

a die Zahlen 0 bis 10 bedeutet,

b, c und d unabhängig voneinander die Zahlen 0 oder 1 bedeuten, wobei, wenn d = 1 ist, die Summe a + b + c ≠ 0 sein muss,

$R_6$ Wasserstoff oder Methyl,

$R_7$ Hydroxy, $C_1$–$C_{12}$ Alkyl, $C_3$–$C_6$ Alkenylmethyl, $C_3$–$C_4$ Alkinylmethyl, $C_7$–$C_{14}$ Aralkyl, Glycidyl, durch Halogen, Cyano, $-COOR_{12}$ oder $-CON(R_{13})(R_{14})$

substituiertes $C_1$–$C_4$ Alkyl, eine Gruppe $-COR_{15}$, $-COOR_{12}$, $-CON(R_{13})(R_{14})$, $-CH_2-CH(R_{16})-OR_{17}$, $-SOR_{18}$, $-SO_2R_{18}$, $-OR_{12}$, $-OCOR_{15}$ ist, wobei $R_{12}$ $C_1$–$C_{12}$ Alkyl, Allyl, Cyclohexyl oder Benzyl, $R_{13}$ $C_1$–$C_{12}$ Alkyl, Allyl, Cyclohexyl, Benzyl, Phenyl oder $C_7$–$C_{10}$ Alkylphenyl, $R_{14}$ Wasserstoff $C_1$–$C_{12}$ Alkyl, Allyl, Cyclohexyl oder Benzyl sind oder $R_{13}$ und $R_{14}$ zusammen mit dem N-Atom an das sie gebunden sind einen 5- oder 6-gliedrigen heterocyclischen Ring bilden, $R_{15}$ Wasserstoff, $C_1$–$C_{12}$ Alkyl, $C_2$–$C_6$ Alkenyl, Chloromethyl, $C_5$–$C_8$ Cycloalkyl, $C_7$–$C_{14}$ Aralkyl, Phenyl, $C_7$–$C_{10}$ Alkylphenyl oder durch 1 oder 2 $C_1$–$C_4$ Alkyl und 1 Hydroxy substituiertes Phenyl, Phenylmethyl oder Phenylethyl, $R_{16}$ Wasserstoff, $C_1$–$C_4$ Alkyl, $C_3$–$C_4$ Alkoxyalkyl, Phenyl oder Phenoxymethyl, $R_{17}$ Wasserstoff, $C_1$–$C_{12}$ Alkyl, eine Gruppe $-COR_{15}$, $-CON(R_{13})(R_{14})$ oder eine Gruppe der Formel

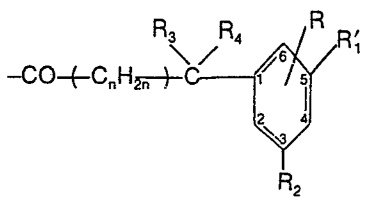

worin $R_1'$ Wasserstoff oder $C_1$–$C_4$ Alkyl ist und $R_{18}$ $C_1$–$C_{12}$ Alkyl, Phenyl oder $C_7$–$C_{10}$ Alkylphenyl bedeuten,
oder $R_7$ eine Gruppe der Formel

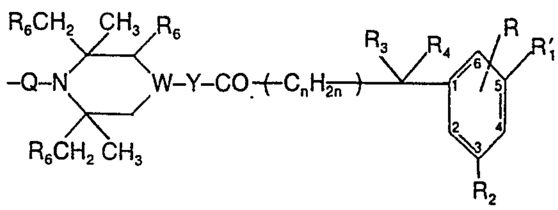

ist, worin
Q $-(-C_rH_{2r}-)-$, worin r die Zahlen 2 bis 12 bedeutet, ist, oder Q ist $C_4$–$C_8$ Alkenylen, $C_5$–$C_{12}$ Cycloalkylen, Phenylen, Xylylen, Bitolylen oder eine Gruppe $-CO-(-C_rH_{2r}-)-CO-$,
Z –O– oder $-N(R_{19})-$ ist, worin $R_{19}$ Wasserstoff, $C_1$–$C_{18}$ Alkyl $C_3$–$C_{12}$ Alkenyl, $C_3$–$C_{12}$ Cycloalkyl, Phenyl, $C_7$–$C_{14}$ Alkaryl, $C_7$–$C_{14}$ Aralkyl, $C_2$–$C_{11}$ Alkoxyalkyl, eine Gruppe $-COR_{20}$, $-COOR_{21}$, $-CON(R_{22})(R_{23})$, $-CH_2-CH(R_{24})-OR_{25}$, $-SOR_{26}$ oder $-SO_2R_{26}$ ist, worin $R_{20}$ eine der für $R_{15}$ angegebenen Bedeutungen hat oder ein heterozyklischer Ring ist, $R_{21}$ eine der für $R_{12}$ angegebenen Bedeutungen hat, $R_{22}$ eine der für $R_{13}$ angegebenen Bedeutungen hat, $R_{23}$ eine der für $R_{14}$ angegebenen Bedeutungen hat, $R_{24}$ eine der für $R_{16}$ angegebenen Bedeutungen hat, $R_{25}$ eine der für $R_{17}$ angegebenen Bedeutungen hat und $R_{26}$ eine der für $R_{18}$ angegebenen Bedeutungen hat, oder $R_{19}$ eine Gruppe der Formel IV ist,
$R_{10}$ Wasserstoff, $C_1$–$C_{18}$ Alkyl, $C_7$–$C_{23}$ Phenoxyalkyl, Phenyl, $C_7$–$C_{14}$ Aralkyl, $C_2$–$C_{11}$ Alkoxyalkyl oder eine Gruppe

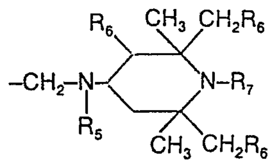

ist,
$R_{11}$ Wasserstoff, $-OR_{27}$, $-OCOR_{28}$, $-N(R_{29})-COR_{28}$, $-OSO_2R_{28}$, $-N(R_{29})-SO_2R_{28}$ ist, wobei $R_{27}$ Wasserstoff, $C_1$–$C_{12}$ Alkyl, Allyl, Benzyl ist, $R_{28}$ Wasserstoff, $C_1$–$C_{12}$ Alkyl, $C_2$–$C_6$ Alkenyl, Chlormethyl, $C_5$–$C_8$ Cycloalkyl, $C_7$–$C_9$ Phenylalkyl, $C_7$–$C_{10}$ Alkylphenyl, Phenyl oder eine Gruppe der Formel

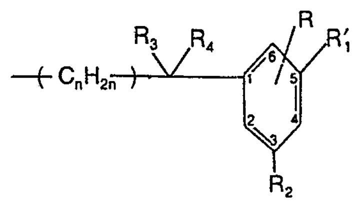

bedeutet und $R_{29}$ Wasserstoff, $C_1$–$C_{12}$ Alkyl, $C_5$–$C_8$ Cycloalkyl oder Benzyl ist und
W eine der Gruppen

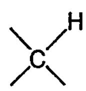

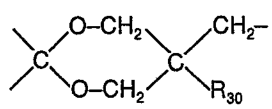

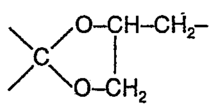

oder

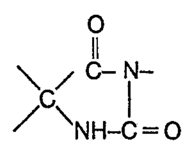

oder
ist, worin $R_{30}$ Methyl oder Ethyl bedeutet mit der Bedingung, dass wenn W eine cyclische Ketalstruktur bedeutet, d = 0 ist und die Summe a + b + c auch 0 sein muss und, wenn W eine Hydantoinstruktur bedeutet, d = 0 und die Summe a + b + c ≠ 0 sein müssen, wobei die wiederholt erwähnten Reste und Symbole immer die erstmals angegebene Bedeutung haben und bei wiederholtem Vorkommen der Gruppe

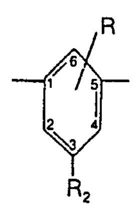

sich R immer in derselben Stellung befindet.

Bedeuten etwaige Substituenten Alkyl, so handelt es sich um geradkettige oder verzweigte Alkylgruppen. Bedeuten sie $C_1$–$C_4$ Alkyl, dann handelt es sich um Methyl, Ethyl, n-Propyl, Isopropyl, n-Butyl, sec.-Butyl oder tert.-Butyl. Bedeuten sie $C_1$–$C_8$ Alkyl, so kommen zusätzlich z.B. n-Pentyl,

2,2-Dimethylpropyl, n-Hexyl, 2,3-Dimethylbutyl, n-Octyl oder 1,1,3,3-Tetramethylbutyl in Frage. Bedeuten sie $C_1$–$C_{12}$ Alkyl, so können sie zusätzlich auch z.B. Nonyl, Decyl, Undecyl und Dodecyl sein. Als $C_1$–$C_{18}$ Alkyl bedeuten sie zusätzlich z.B. Tetradecyl, Hexadecyl, Heptadecyl oder Octadecyl. Bedeuten etwaige Substituenten $C_5$–$C_8$ Cycloalkyl, so handelt es sich z.B. um Cyclopentyl, Cyclohexyl, Cycloheptyl, α-Methylcyclohexyl, Cyclooctyl oder Dimethylcyclohexyl. Als $C_3$–$C_{12}$ Cycloalkyl können sie zusätzlich z.B. Cyclopropyl, Cyclononyl, Cyclodecyl oder Cyclododecyl sein. Bevorzugt ist Cyclohexyl.

$R_{28}$ bedeutet als $C_7$–$C_9$ Phenylalkyl beispielsweise Benzyl, Phenylethyl oder Phenylpropyl. Bedeuten etwaige Substituenten $C_7$–$C_{14}$ Aralkyl, so handelt es sich darüber hinaus auch z.B. um Phenylbutyl oder Naphthylmethyl.

Bedeuten etwaige Substituenten $C_7$–$C_{10}$ Alkylphenyl, so können sie beispielsweise Tolyl, Xylyl, Isopropylphenyl, tert.-Butylphenyl oder Diethylphenyl sein.

$R_7$ ist als $C_3$–$C_6$ Alkenylmethyl z.B. Allyl, Methallyl, Dimethylallyl oder 2-Hexenyl. $R_{15}$ und $R_{28}$ können als $C_2$–$C_6$ Alkenyl zusätzlich auch Vinyl sein.

$R_5$ und $R_{19}$ können als $C_3$–$C_{12}$ Alkenyl beispielsweise Allyl, Methallyl, 2-Butenyl, 2-Hexenyl, 2-Octenyl, 4-Octenyl, 2-Decenyl oder 2-Dodecenyl sein. Bevorzugt ist Allyl.

$R_7$ bedeutet als $C_3$–$C_4$ Alinylmethyl z.B. Propargyl, n-But-1-inyl oder n-But-2-inyl. Bevorzugt ist Propargyl.

Bedeuten etwaige Substituenten $C_7$–$C_{14}$ Alkaryl so handelt es sich z.B. um durch $C_1$–$C_4$ Alkyl substituiertes Phenyl, wie p-Tolyl, 2,4-Dimethylphenyl, 2,6-Dimethylphenyl, 2,4-Diethylphenyl, 2,6-Diethylphenyl, 4-tert.-Butylphenyl, 2,4-Di-tert.-butylphenyl oder 2,6-Di-tert.-butylplhenyl. Bevorzugt sind 2,4-Di-tert.-butylphenyl und 2,4-Dimethylphenyl.

$R_{15}$ als heterozyklischer Ring bedeutet beispielsweise Pyrrol, Pyridin, Indol, Chinolin, Pyrrolidin, Thiophen, Furan, Imidazol, Pyrazin, Pyrimidin, Thiazol, Oxazol, Piperazin, Morpholin oder Piperidin.

$R_{16}$ bedeutet als $C_3$–$C_4$ Alkoxyalkyl, z.B. Ethoxymethyl, 2-Methoxyethyl oder 2-Ethoxyethyl. $R_5$, $R_{10}$ und $R_{19}$ können als $C_2$–$C_{11}$ Alkoxyalkyl darüber hinaus auch Methoxymethyl, 2-n-Butoxyethyl, 2-n-Butoxypropyl, 2-n-Octoxyethyl, 3-n-Octoxypropyl oder 6-n-Butoxyphenyl sein.

$R_{10}$ ist als $C_7$–$C_{23}$ Phenoxyalkyl z.B. Phenoxymethyl, Phenoxyethyl, Phenoxypropyl, Phenoxybutyl, Phenoxyoctyl, Phenoxydecyl, Phenoxydodecyl oder Phenoxyhexadecyl.

Bedeuten etwaige Substituenten Halogen, so handelt es sich z.B. um Brom, Jod und insbesondere Chlor.

Bei der Gruppe $-C_nH_{2n}-$, worin n eine Zahl zwischen 1 und 20 ist, bedeutet n bevorzugt eine Zahl zwischen 2 und 8 und insbesondere 3. Beispiele sind Methylen, Äthylen, Trimethylen, Tetramethylen, Hexamethylen, Octamethylen, Nonamethylen, 2,2,4-Trimethylhexamethylen, Decamethylen, Dodecamethylen.

$R_{15}$ und $R_{20}$ bedeuten als durch 1 oder 2 $C_1$–$C_4$ Alkyl und 1 Hydroxy substituiertes Phenyl, Phenylmethyl oder Phenylethyl z.B. 2,5-Dimethyl-4-hydroxyphenyl, 3,5-Di-tert.butyl-4-hydroxyphenyl, 3,5-Dimethyl-4-hydroxybenzyl, 3,5-Di-tert.butyl-4-hydroxybenzyl oder 2-(3,5-Di-tert.-butyl-4-hydroxyphenyl)-ethyl.

Bevorzugt werden farbphotographische Aufzeichnungsmaterialien, die als Stabilisatoren mindestens eine Verbindung der Formel Ia enthalten

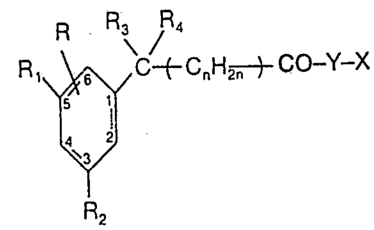

(Ia),

worin die Substituenten R, $R_1$, $R_2$, $R_3$, $R_4$, Y und X, sowie der Index n, die schon oben definierte Bedeutung besitzen.

Bevorzugt werden solche farbphotographischen Aufzeichnungsmaterialien, die als Stabilisator mindestens eine Verbindung der Formel V

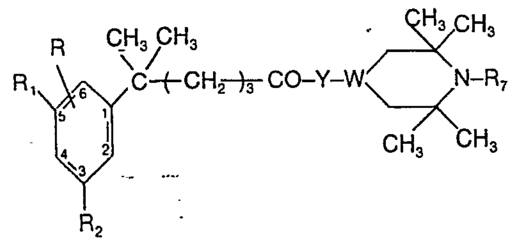

(V)

enthalten, worin

R eine OH-Gruppe ist, die sich in 2-, 4- oder 6-Stellung befindet,

$R_1$ Wasserstoff, $C_1$–$C_4$ Alkyl, eine Gruppe der Formel VI

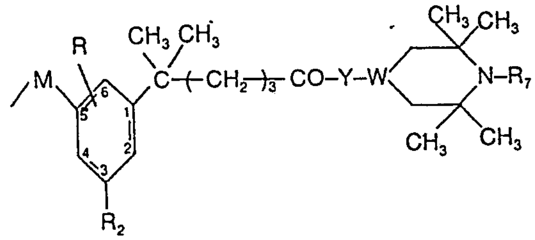

(VI)

oder eine Gruppe der Formel VII

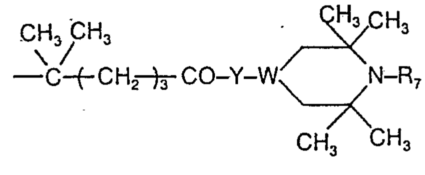

(VII)

ist,

$R_2$ $C_1$–$C_4$ Alkyl oder eine Gruppe der Formel VII bedeutet

Y –O– oder –N($R_5$)–, worin $R_5$ Wasserstoff, $C_1$–$C_{12}$ Alkyl, Cyclohexyl, $C_3$–$C_4$ Alkoxyalkyl oder eine Gruppe der Formel VIII

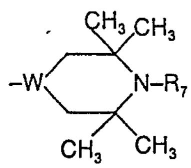

(VIII)

ist, bedeutet,

M –CH($R_8$)– oder –S– ist, wobei $R_8$ Wasserstoff oder $C_1$–$C_4$ Alkyl bedeutet,

$R_7$ Hydroxy, Methyl, Allyl, Benzyl, 2-Hydroxyethyl, Acetyl, Acroyl oder eine Gruppe –CON($R_{13}$)($R_{14}$), worin $R_{13}$ $C_1$–$C_8$ Alkyl, Cyclohexyl oder Phenyl und $R_{14}$ Wasserstoff, $C_1$–$C_8$ Alkyl oder Cyclohexyl bedeuten, ist oder

$R_7$ eine Gruppe der Formel

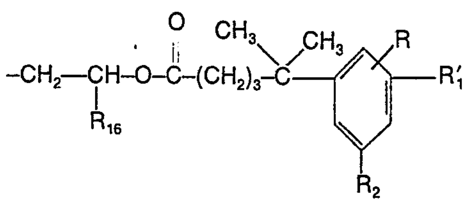

ist, worin $R_{16}$ Wasserstoff oder Methyl bedeutet und

W eine der Gruppen

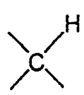

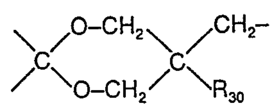

oder

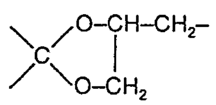

ist, worin $R_{30}$ Methyl oder Ethyl bedeutet, wobei die in dieser Bevorzugung wiederholt erwähnten Reste immer die in dieser Bevorzugung erstmals angegebene Bedeutung haben und bei wiederholtem Vorkommen der Gruppe

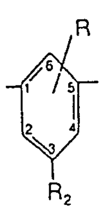

sich R immer in derselben Stellung befindet.

Besonders bevorzugt werden farbphotographische Aufzeichnungsmaterialien, die als Stabilisator mindestens eine Verbindung der Formel IX

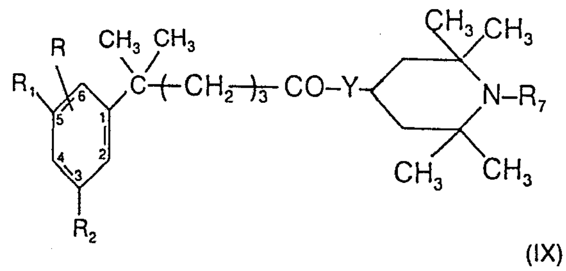

(IX)

enthalten, worin

R eine OH-Gruppe ist, die sich in 2-, 4- oder 6-Stellung befindet,

$R_1$ Wasserstoff, $C_1$–$C_4$ Alkyl oder eine Gruppe der Formel X

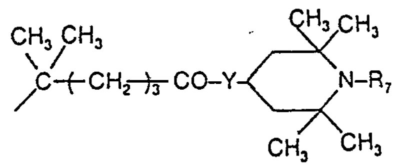

(X)

$R_2$ $C_1$–$C_4$ Alkyl oder eine Gruppe der Formel X

Y –O– oder –N($R_5$)–, worin $R_5$ Wasserstoff oder $C_1$–$C_8$ Alkyl bedeutet und

$R_7$ Hydroxy, Methyl, Allyl, Benzyl, 2-Hydroxyethyl, Acetyl, Acryloyl oder eine Gruppe der Formel

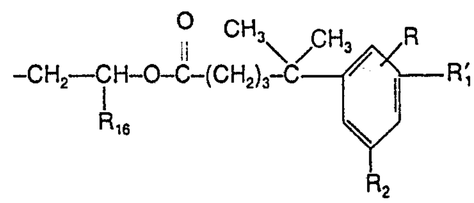

ist, worin $R_{16}$ Wasserstoff oder Methyl bedeutet.

Bevorzugt werden weiterhin farbphotographische Aufzeichnungsmaterialien, die als Stabilisator mindestens eine Verbindung der Formel I oder der Formel V oder der Formel IX enthalten, bei der sich der Rest R in 2- oder in 4- oder in 6-Position befindet.

Die Verbindungen der Formel I sind neu und sind für sich ebenfalls Gegenstand der vorliegenden Erfindung.

Sie können in Analogie zu bekannten Verbindungen erhalten werden, wie sie z.B. in den DE-OS 2 456 364, 2 647 452, 2 654 058 und 2 656 769 beschrieben sind. Die letzte Stufe der Synthese ist entweder eine direkte Veresterung (Säure + Alkohol oder Säurechlorid + Alkohol), eine Umesterung oder eine Amidierung. Die Polyalkylpiperidinverbindungen, die als Ausgangsprodukt für die Herstellung der Verbindungen der Formel I eingesetzt werden, sind bekannt. Sollten einzelne von ihnen noch neu sein; so können sie in Analogie zu den bekannten erhalten werden.

Die als Ausgangsprodukte verwendeten sterisch gehinderten Phenolderivate sind neu. Es handelt sich dabei um Phenole der Formel XI

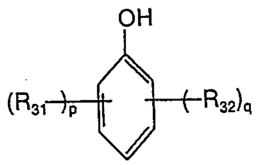

(XI)

worin p die Zahlen 1, 2 oder 3 und q die Zahlen 0, 1 oder 2 bedeuten mit der Bedingung, dass $p + q \leq 3$ ist, $R_{31}$ eine Gruppe der Formel XII

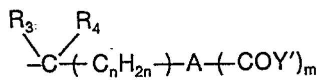

(XII)

ist, worin $R_3$, $R_4$, A, m und n die oben angegebene Bedeutung haben und Y′ –OR′ oder $-N(R_5)H$ ist, wobei R′ Wasserstoff, Methyl oder Ethyl bedeutet und $R_5$ die oben angegebene Bedeutung hat, und $R_{32}$ $C_1$–$C_4$ Alkyl oder eine der Gruppen

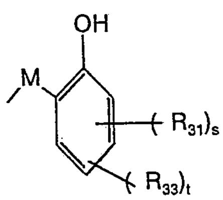

oder

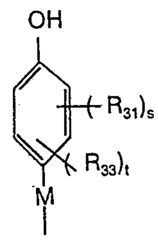

(XIII) (XIV)

die in 2-, 4- oder 6-Stellung zur OH-Gruppe steht, ist, worin M und $R_{31}$ die oben angegebene Bedeutung haben, $R_{33}$ $C_1$–$C_4$ Alkyl bedeutet, s die Zahlen 0, 1 oder 2 und t die Zahlen 0 oder 1 bedeuten, mit der Bedingung, dass $s + t \leq 2$ ist; wobei stets die Bedingung gilt, dass m 2 sein muss, wenn n 1 oder 2 ist und wenn R′ Wasserstoff bedeutet, wobei im Phenol der Formel XI nicht mehr als eine Gruppe der Formel XIII oder XIV vorkommen kann.

Die Phenole der Formel XI können durch Umsetzung eines Phenols der Formel XV

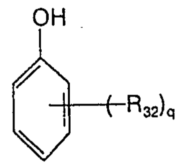

XV

worin $R_{32}$ und q die oben angegebene Bedeutung haben und mit der Bedingung, dass eine Gruppe der Formel XIII oder XIV nicht mehr als einmal in der Formel XV vorkommen darf, und zwar in 2-, 4- oder 6-Stellung zur OH-Gruppe, mit einem funktionalen Alkylierungsmittel (XXI), das zur Einführung einer Gruppe der Formel XII befähigt ist, in Anwesenheit eines geeigneten Katalysators, hergestellt werden.

Die Alkylierung erfolgt bei Temperaturen zwischen 20 und 170, bevorzugt zwischen 100 und 150 °C. Geeignete Katalysatoren sind Brönsted-Säuren, aktive Erden oder Metallsalze. Als Brönsted-Säuren kommen organische oder anorganische Säuren oder auch deren Teilsalze in Frage. Es kann sich z. B. um eine Mineralsäure wie Salz-, Schwefel-, Perchlor- oder Orthophosphorsäure, um eine durch Alkyl, Aryl oder Alkaryl substituierte anorganische Säure wie Methan-, Ethan-, Benzol-, p-Toluolsulfonsäure oder Methanphosphonsäure oder um eine organische Säure wie Dichlor-, Trichlor- oder Trifluoressigsäure handeln. Geeignete aktive Erden sind z. B. Fulmont 237® oder Fulcat 22®, während als Metallsalz beispielsweise Aluminiumphenoxyd in Frage kommt. Bevorzugt sind die aktiven Erden.

Die Reaktion kann mit oder ohne Lösungsmittel durchgeführt werden. Geeignete Lösungsmittel sind z. B. Methanol/Schwefelsäure oder Wasser/Schwefelsäure. Diese Lösungsmittel wirken auch als Katalysatoren. Die Verbindungen der Formel XI erhält man aus Verbindungen der Formel XV durch Umsetzung mit 0,1 bis 4,0 Molen des Alkylierungsmittels (XXI), je nach der Bedeutung von p und q.

Typische Vertreter von Verbindungen der Formel I sind in den nachstehenden Tabellen I bis VI aufgeführt.

Tabelle I

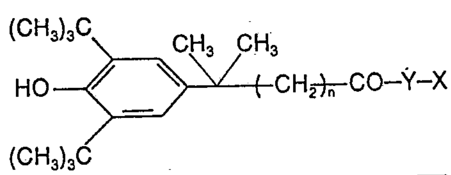

| Stabilisator Nr. | n | Y | X |
|---|---|---|---|
| 1 | 3 | –O– | 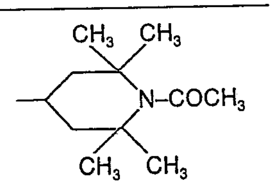 |

Tabelle I (Fortsetzung)

| Stabilisator Nr. | n | Y | X |
|---|---|---|---|
| 2 | 3 | –O– | 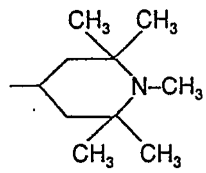 |
| 3 | 3 | –O– | 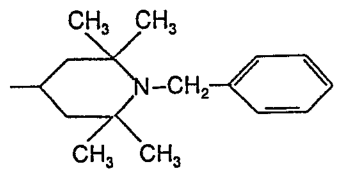 |
| 4 | 3 | –O– | 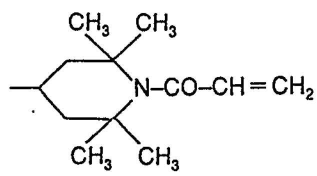 |
| 5 | 3 | –O– | 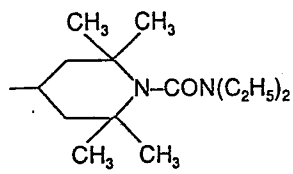 |
| 6 | 3 | –O– | 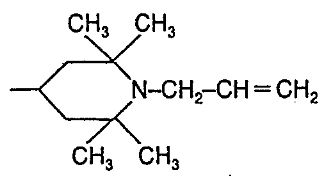 |
| 7 | 1 | –O– | 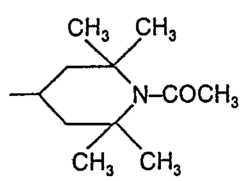 |
| 8 | 3 | –O– | 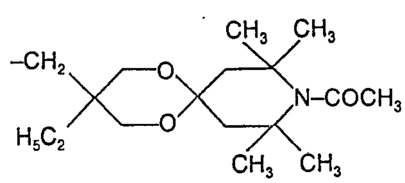 |
| 9 | 3 | –NH– | 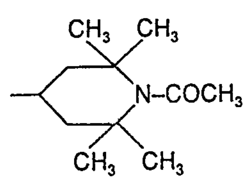 |

Tabelle I (Fortsetzung)

| Stabilisator Nr. | n | Y | X |
|---|---|---|---|
| 10 | 3 | $-NH-$ | 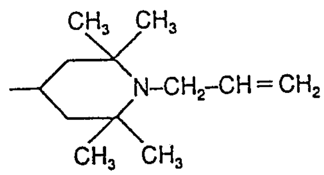 |
| 11 | 3 | $-N(CH_3)-$ | 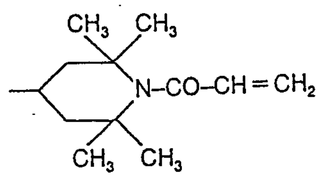 |
| 12 | 1 | $-N(CH_3)-$ | 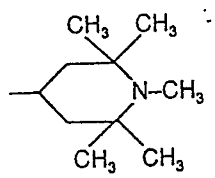 |
| 13 | 3 | 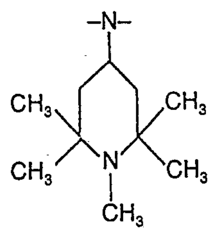 | 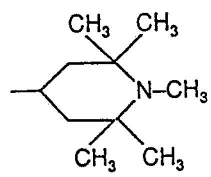 |

Tabelle II

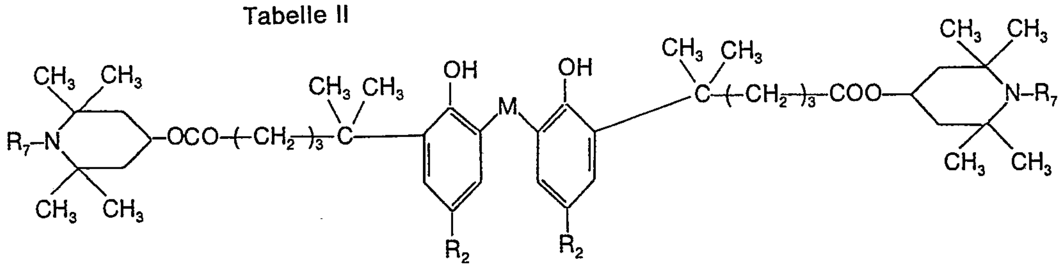

| Stabilisator Nr. | M | $R_2$ | $R_7$ |
|---|---|---|---|
| 14 | $-CH_2-$ | $-CH_3$ | $-COCH_3$ |
| 15 | $-CH_2-$ | $-C(CH_3)_3$ | $-COCH_3$ |
| 16 | $-CH_2-$ | $-CH_3$ | $-CO-CH=CH_2$ |
| 17 | $-CH_2-$ | $-CH_3$ | $-CH_3$ |
| 18 | $-CH_2-$ | $-CH_3$ | 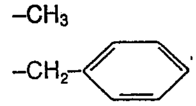 |
| 19 | $-S-$ | $-C(CH_3)_3$ | $-COCH_3$ |
| 20 | $-S-$ | $-CH_3$ | $-CH_3$ |
| 21 | $-S-$ | $-CH_3$ | 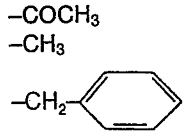 |
| 22 | $-S-$ | $-C(CH_3)_3$ | $-CO-CH=CH_2$ |

Tabelle III

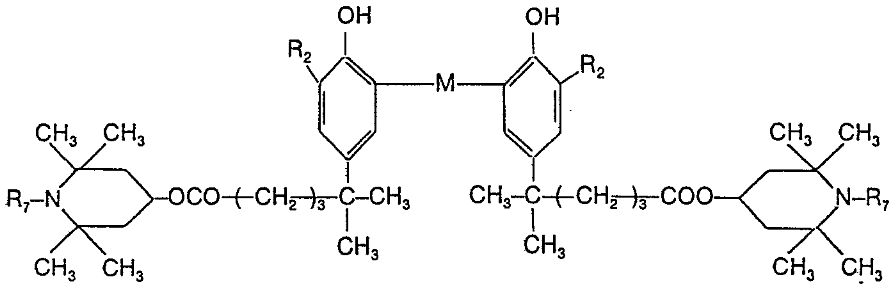

| Stabilisator Nr. | M | $R_2$ | $R_7$ |
|---|---|---|---|
| 23 | – | $-C(CH_3)_3$ | $-CH_3$ |
| 24 | – | $-C(CH_3)_3$ | $-COCH_3$ |
| 25 | $-CH_2-$ | $-C(CH_3)_3$ | $-CH_2-$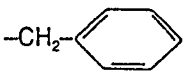 |
| 26 | –S– | $-C(CH_3)_3$ | $-CH_3$ |

Tabelle IV

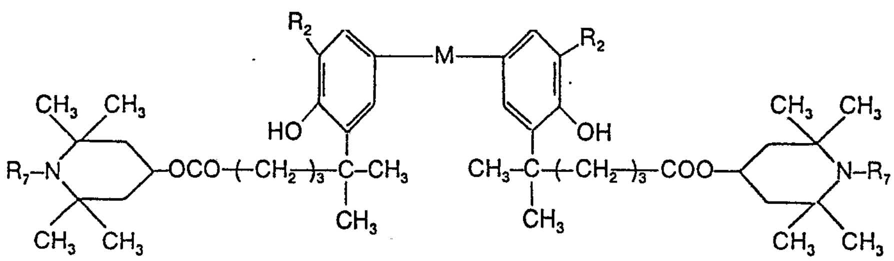

| Stabilisator Nr. | M | $R_2$ | $R_7$ |
|---|---|---|---|
| 27 | – | $-C(CH_3)_3$ | $-COCH_3$ |
| 28 | $-CH_2-$ | $-CH_3$ | $-CO-CH=CH_2$ |
| 29 | –S– | $-C(CH_3)_3$ | $-COCH_3$ |

Tabelle V:

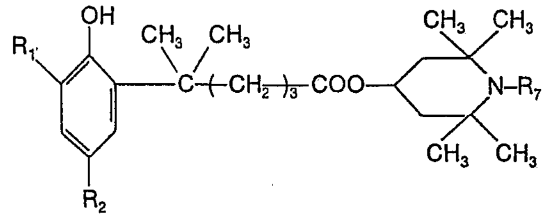

| Stabilisator Nr. | $R_1$ | $R_2$ | $R_7$ |
|---|---|---|---|
| 30 | 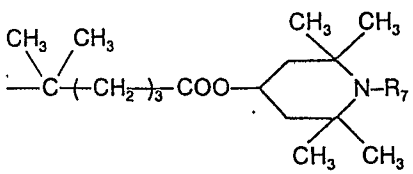 | 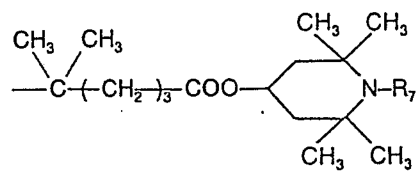 | $-COCH_3$ |
| 31 | 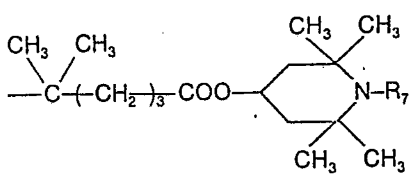 | 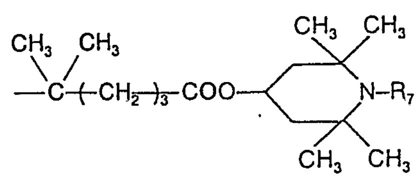 | 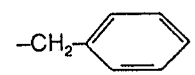 |
| 32 | 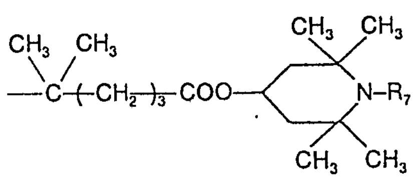 | $-CH_3$ | $-COCH_3$ |
| 33 | $-H$ | $-C(CH_3)_3$ | $-CO-CH=CH_2$ |
| 34 | $-C(CH_3)_3$ | $-C(CH_3)_3$ | $-COCH_3$ |
| 35 | $-C(CH_3)_3$ | $-C(CH_3)_3$ | $-CON(C_2H_5)_2$ |

Tabelle VI:

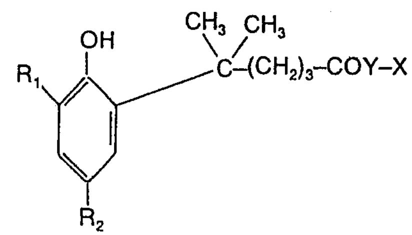

| Stabili-sator Nr. | $R_1$ | $R_2$ | Y | X |
|---|---|---|---|---|
| 36 | $-C(CH_3)_3$ | $-C(CH_3)_3$ | –O– | 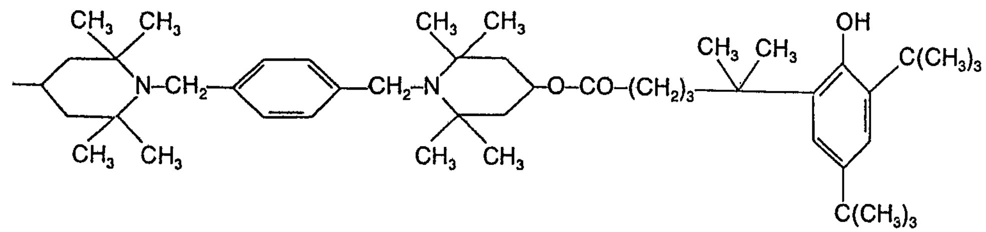 |
| 37 | $-C(CH_3)_3$ | $-C(CH_3)_3$ | –O– | 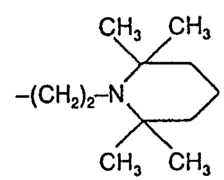 |
| 38 | $-C(CH_3)_3$ | $-C(CH_3)_3$ | –O– | 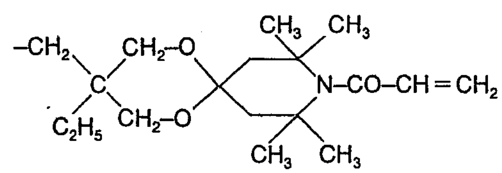 |

Tabelle VI: (Fortsetzung)

| Stabilisator Nr. | $R_1$ | $R_2$ | Y | X |
|---|---|---|---|---|
| 39 | $-C(CH_3)_3$ | $-C(CH_3)_3$ | –O– | 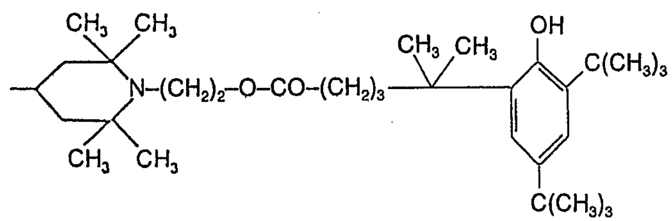 |
| 40 | H | $-C(CH_3)_3$ | –NH– | 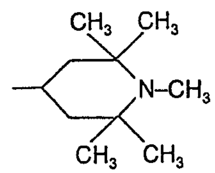 |

Weitere typische Vertreter von Verbindungen der Formel I sind die folgenden:

41.

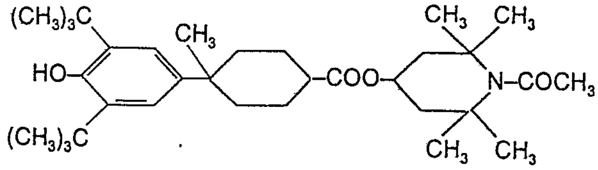

42.

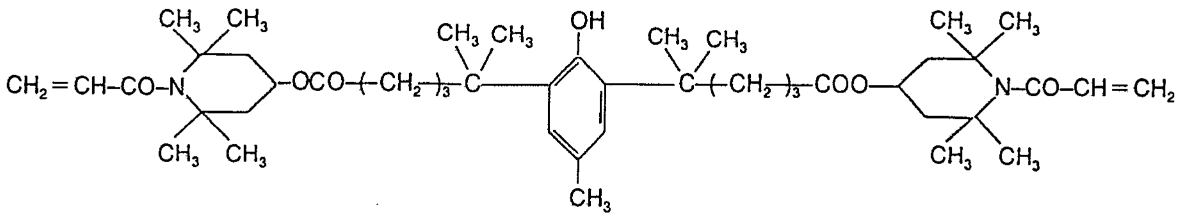

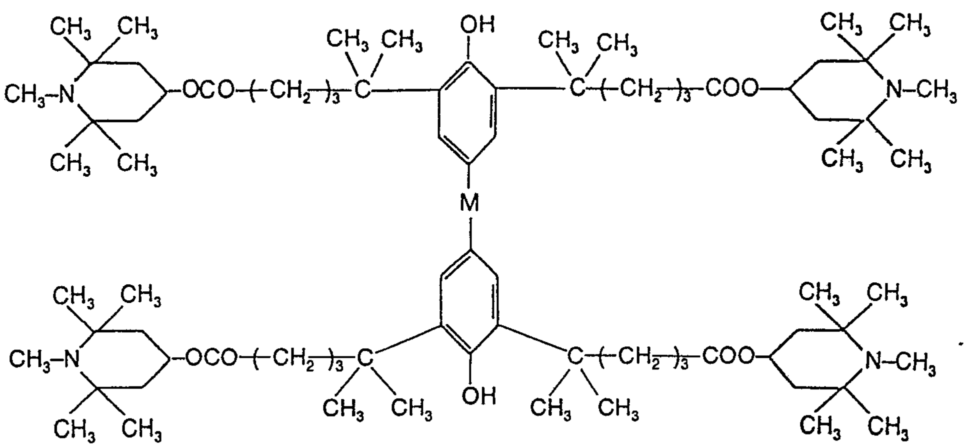

43. M = $-CH_2-$

44. M = $-S-$

45.

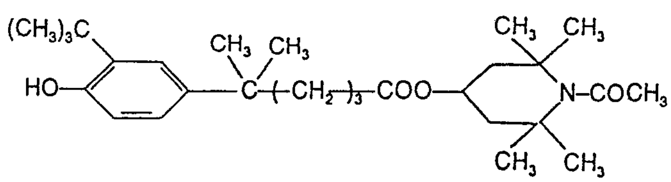

46.

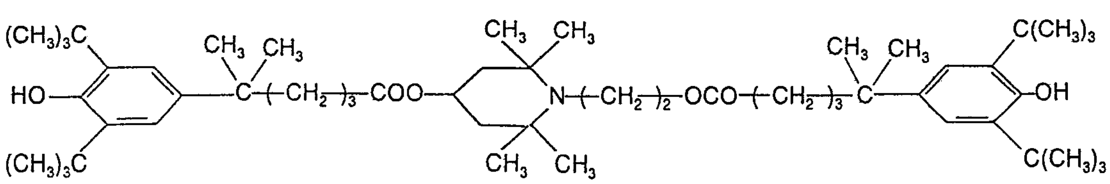

47.
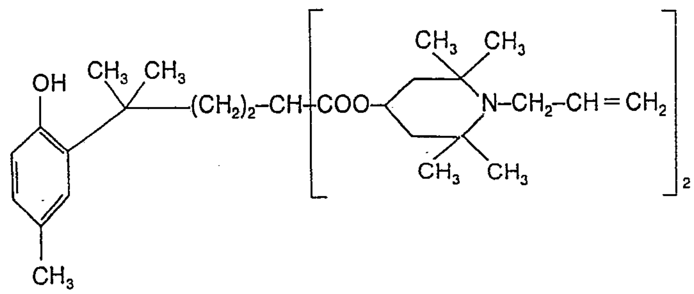

48.
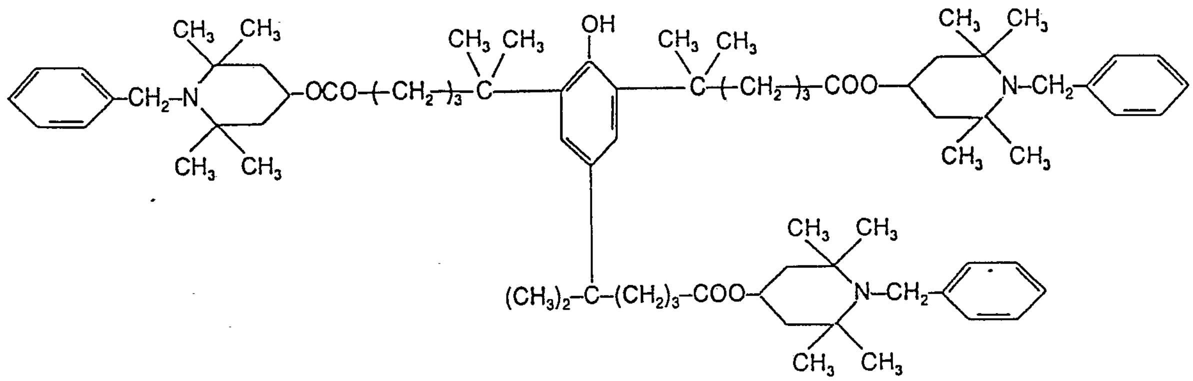

49.
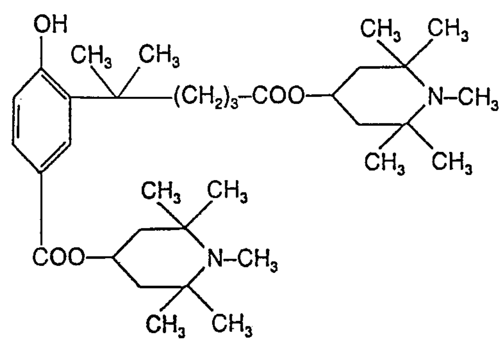

Die Stabilisatoren der Formel I können allein oder zusammen mit anderen Verbindungen in bekannter Weise in ein photographisches Material eingearbeitet werden.

In der Regel werden die Stabilisatoren allein oder zusammen mit anderen Verbindungen, insbesondere mit Farbkupplern, in Form einer Dispersion in das photographische Material eingearbeitet, wobei diese Dispersion entweder kein Lösungsmittel oder hoch- oder tiefsiedende Lösungsmittel oder ein Gemisch solcher Lösungsmittel enthält. Eine weitere geeignete Einarbeitungsform besteht darin, dass man die Stabilisatoren allein oder zusammen mit weiteren Verbindungen zusammen mit einem Polymer in Form eines Latex in das photographische Material einarbeitet.

Die Dispersionen werden dann zur Herstellung der Schichten von farbphotographischen Aufzeichnungsmaterialien verwendet. Diese Schichten können z.B. Zwischen- oder Schutzschichten, insbesondere jedoch lichtempfindliche (blau-, grün- und rotempfindliche) Silberhalogenidemulsionsschichten sein, in denen bei der Entwicklung des belichteten Aufzeichnungsmaterials aus den entsprechenden Farbkupplern, die Blaugrün (Cyan)-, Purpur (Magenta)- und Gelbfarbstoffe gebildet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Da das Substrat die Wirkung und Stabilität der Verbindungen der Formel I beeinflusst, werden Substrate (Lösungsmittel, Polymere) bevorzugt, die zusammen mit diesen Stabilisatoren eine möglichst gute Beständigkeit der zu stabilisierenden Materialien ergeben.

In der Regel werden die Stabilisatoren der Formel I in Schichten eingearbeitet, die zusätzlich eine nach üblichen Methoden hergestellte und sensibilisierte Silberhalogenid-Dispersion enthalten. Sie können jedoch auch in zu Silberhalogenid enthaltenden Schichten benachbarten Schichten vorhanden sein.

Die erfindungsgemässen photographischen Materialien besitzen einen üblichen Aufbau und

übliche Komponenten. Bevorzugt werden jedoch ein Aufbau und Komponenten, die die Wirksamkeit der Stabilisatoren der Formel I verstärken oder zumindest nicht nachteilig beeinflussen.

Im photographischen Aufzeichnungsmaterial gemäss vorliegender Erfindung können die Stabilisatoren gemäss Formel I, ausser mit den Farbkupplern zusätzlich auch mit Ultraviolettabsorbern oder anderen Lichtschutzmitteln in der gleichen Schicht kombiniert werden.

Wenn die Diffusionstransfermethode angewandt wird, kann der Stabilisator auch in eine Empfangsschicht eingearbeitet werden.

Die erfindungsgemässen farbphotographischen Materialien können in bekannter Weise verarbeitet werden. Ferner können sie im Verlauf oder nach der Verarbeitung in einer Weise behandelt werden, die ihre Stabilität weiter erhöht, beispielsweise durch die Behandlung in einem Stabilisatorbad oder das Aufbringen eines Schutzüberzuges.

Die erfindungsgemäss einzusetzenden Stabilisatoren eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2000 mg, vorzugsweise 100 bis 800 und insbesondere 200–500 mg pro $m^2$ der Schicht, in die es (sie) eingearbeitet wird (werden).

Falls das photographische Material ein UV-Strahlung absorbierendes Mittel enthält, so kann dieses mit dem Stabilisator zusammen in einer Schicht oder auch in einer benachbarten Schicht vorhanden sein. Die Menge des UV-Absorbers oder der UV-Absorber kann in weiten Grenzen schwanken und liegt etwa im Bereich von 200 bis 2 000 mg, vorzugsweise 400 bis 1 000 mg pro $m^2$ der Schicht, in der er (sie) eingearbeitet wird (werden). Beispiele für Ultraviolett-Absorber sind Verbindungen vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formel I sind praktisch farblos, so dass es zu keiner Verfärbung der Bilder kommt; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässrigen Bindemittelemulsionen, die für die Herstellung photographischer Schichten verwendet werden, einarbeitet. Die einzelnen nach der Belichtung des photographischen Auszeichnungsmaterials notwendigen Verarbeitungsschritte zur Herstellung der Farbbilder werden durch die Stabilisatoren der Formel I nicht nachteilig beeinflusst. Ferner kann die bei blauempfindlichen Emulsionen häufig auftretende sogenannte Druckschleierbildung weitgehend zurückgedrängt werden. Diese kann z.B. auftreten, wenn auf photographische Materialien (Silberhalogenidemulsionsschichten, die auf einem Träger aus natürlichen oder synthetischen Materialien befinden) mechanische Beanspruchungen, z.B. Drehen, Biegen oder Reiben, während der Herstellung oder während der Behandlung vor der Entwicklung ausgeübt werden. (T.H. James, The Theory of Photographic Process 4. Auflage, Macmillan, New York, N.Y. 1977, Seite 23ff., S. 166ff.).

Die nachfolgenden Beispiele dienen der näheren Erläuterung der Erfindung. Teile sind hierin Gewichtsteile.

Herstellung der phenolischen Ausgangsprodukte

Beispiel 1:

94 Teile Phenol, 14,2 Teile Methyl-5-methyl-hex-5-enoat und 5,0 Teile Fulmot 237® werden 20 Stunden bei 110 °C gerührt. Aus dem teilweise abgekühlten Reaktionsgemisch wird der Katalyator abfiltriert. Nach Abtrennung von 82 Teilen Phenol erhält man das Methyl-5-(4-hydroxyphenyl)-5-methyl-hexanoat mit Siedepunkt bei $T_{0,65\ mbar}$ von 167–172 °C.

Analyse für $C_4H_{20}O_3$

| | C | H |
|---|---|---|
| Gefunden | 71,01 % | 8,60 % |
| Berechnet | 71,16 % | 8,83 % |

Verfährt man wie unter Beispiel 1 beschrieben unter den in der nachstehenden Tabelle VII angegebenen Bedingungen, so erhält man die in Tabelle VII aufgeführten sterisch gehinderten Phenole.

Tabelle VII:

| Beispiel Nr. | Phenol (Teile) | Alkylierungsmittel (Teile) | Katalysator (Teile) | Reaktions-Temp. in °C | Reaktions-zeit in Stunden | Endprodukt |
|---|---|---|---|---|---|---|
| 2 | 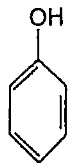 (94) | 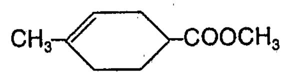 (30,8) | Al(OPh)$_3$ von Al(0,3) | 175 | 40 | 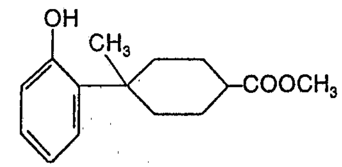 |
| 3 | 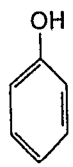 (37,8) | 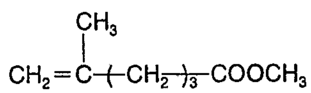 (28,4) | Fulmont 237® (5,0) | 125 | 20 | 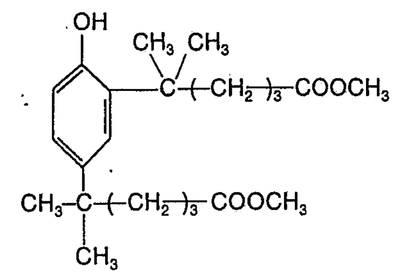 |
| 4 | 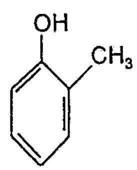 (108) | 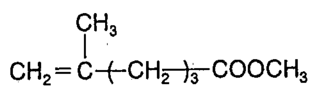 (56,8) | Fulmont 237® (5,0) | 125 | 24 | 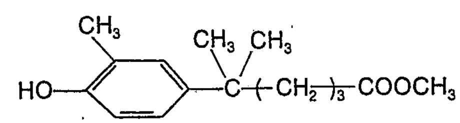 |
| 5 | 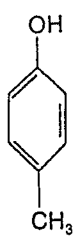 (108) | 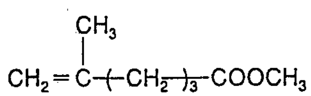 (56,8) | Fulmont 237® (5,0) | 150 | 20 | 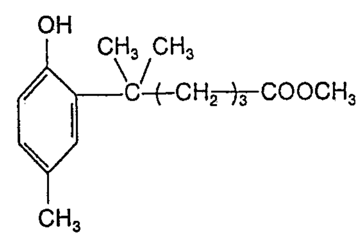 |

Tabelle VII: (Fortsetzung)

| Beispiel Nr. | Phenol (Teile) | Alkylierungsmittel (Teile) | Katalysator (Teile) | Reaktions-Temp. in °C | Reaktions-zeit in Stunden | Endprodukt |
|---|---|---|---|---|---|---|
| 6 | 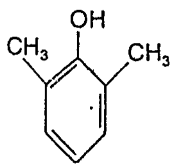 (122) | 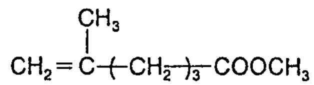 (28,4) | Fulmont 237® (10) | 120 | 24 | 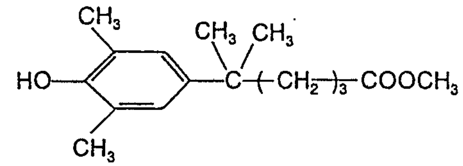 |
| 7 | 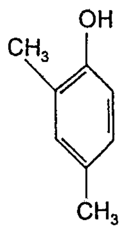 (122) | 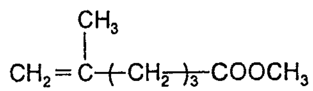 (28,4) | Fulmont 237® (10) | 120 | 24 | 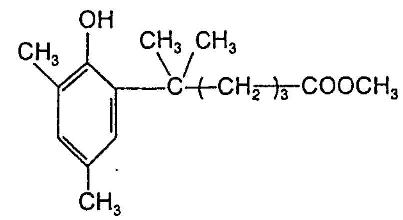 |
| 8 | 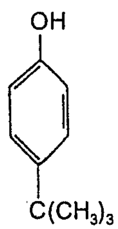 (75) | 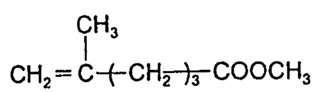 (28,4) | Fulmont 237® (5,0) | 125 | 20 | 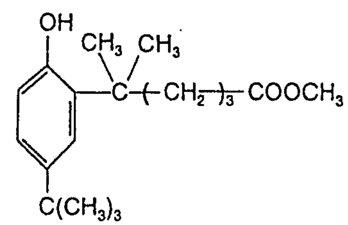 |

Beispiel 9:

12,5 Teile Methyl-5-(2-hydroxy-5-methylphenyl)-5-methylhexanoat (hergestellt gemäss Beispiel 5), 14,2 Teile Methyl-5-methylhex-5-enoat und 1,0 Teil p-Toluolsulfonsäure werden 8 Tage auf Wasserdampf erhitzt. Das Reaktionsgemisch wird dann mit Ether verdünnt, mit 2N-Natronlauge und dann mit Wasser gewaschen und schliesslich eingedampft. Nach dem Destillieren im Vakuum des zurückgebliebenen Öls und nach Umkristallisieren des Destillats in Petrolether erhält man Bis-2,6-(5-methoxycarbonyl-2-methyl-pent-2-yl)-4-methyl-phenol mit Smp. 55–57 °C.

Analyse für $C_{23}H_{36}O_5$

| | C | H |
|---|---|---|
| Gefunden | 70,25 % | 9,01 % |
| Berechnet | 70,38 % | 9,24 % |

Herstellung der erfindungsgemäss einzusetzenden Stabilisatoren der Formel I

Durch Umesterung nach üblichen Methoden der Endprodukte der Beispiele 1 bis 9 mit den Polyalkylpiperidinverbindungen

a) 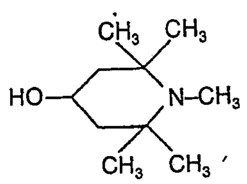

b) 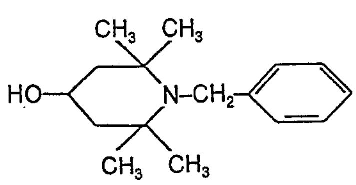

c) 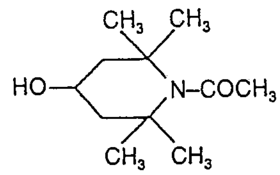

d) 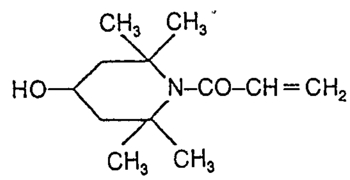

e) 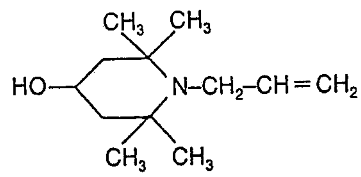

f) 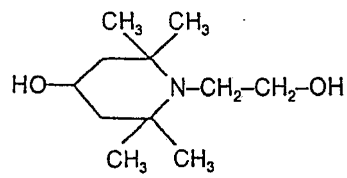

g) 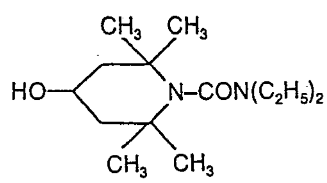

erhält man die entsprechenden erfindungsgemäss einzusetzenden Stabilisatoren.

Anwendungsbeispiele

0,087 g des Gelbkupplers der Formel

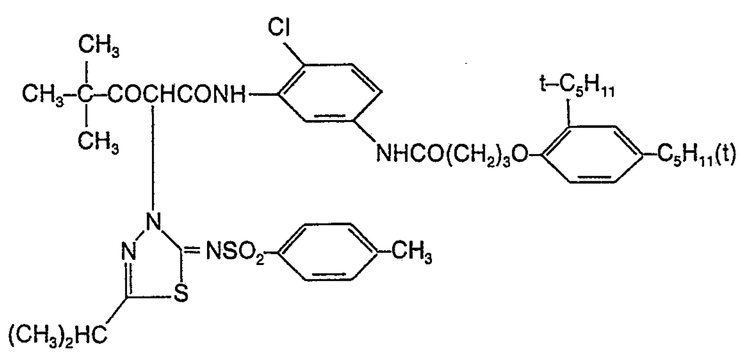

und 0,026 g eines der in den nachfolgenden Tabellen angegebenen Lichtschutzmittels werden in 2,0 ml eines Gemisches von Trikresylphosphat/Äthylacetat (1,5 g in 100 ml) gelöst. Zu dieser Lösung gibt man 7.0 ml einer 6%-igen Gelatinelösung, 0,5 ml einer 8%-igen Lösung des Netzmittels der Formel

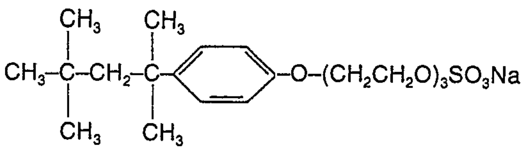

in Isopropanol/Wasser (3:4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1%-igen wässerigen Lösung des Härters der Formel

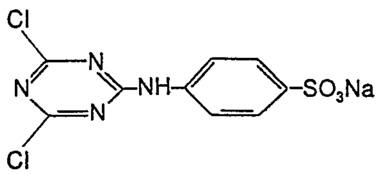

und 3,8 ml Wasser, stellt das Gemisch auf einen pH-Wert von 6,5 und vergiesst es auf eine Glasplatte aufgezogenes substriertes, kunststoffbeschichtetes, weisses Papier.

Nach dem Erstarren wird in einem Trockenschrank mit Umluft bei Raumtemperatur getrocknet.

Nach 7 Tagen werden 35 × 180 mm geschnittene Proben hinter einem Stufenkeil mit 3000 Lux · s belichtet und anschliessend im Ektaprint 2®-Prozess der Firma Kodak verarbeitet.

Die so erhaltenen Gelbkeile werden in einem Atlas Weather-Ometer mit einer 2500 W-Xenonlampe mit total 42 kJoule/cm$^2$ bestrahlt (eine Vergleichsprobe enthält kein Lichtschutzmittel).

Der dabei eingetretene Farbdichteverlust wird bestimmt durch Messung der Farbdichte bei λ max. mit einem Densitometer® TR 924A der Firma Macbeth.

Die Ergebnisse sind in der folgenden Tabelle aufgeführt:

| Lichtschutzmittel Nr. | Verlust am Maximum in Remission in Prozenten |
|---|---|
| – | 36 |
| 14 | 16 |
| 40 | 21 |
| 42 | 21 |
| 47 | 15 |

**Patentansprüche**

1. Farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Stabilisator enthält, dadurch gekennzeichnet, dass die Polyalkylpiperidinverbindung der Formel I

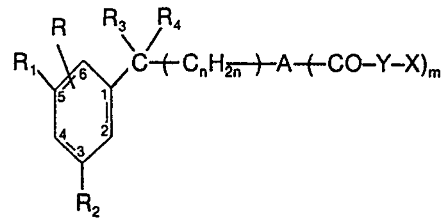 (I)

entspricht, worin

R eine OH-Gruppe ist, die sich in 2-, 4- oder 6-Stellung befindet,

$R_1$ Wasserstoff, $C_1$–$C_4$ Alkyl, eine Gruppe der Formel II

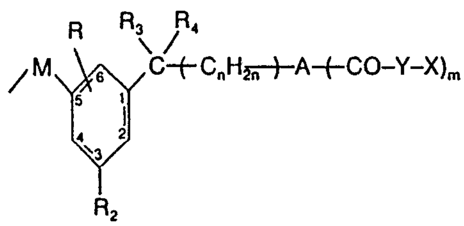 (II)

oder eine Gruppe der Formel III

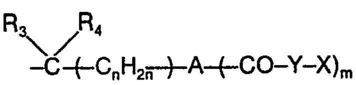 (III)

ist,

$R_2$ $C_1$–$C_4$ Alkyl, eine Gruppe der Formel III oder eine Gruppe der Formel –CO–Y–X bedeutet,

$R_3$ und $R_4$ unabhängig voneinander $C_1$–$C_8$ Alkyl sind und $R_4$ zusätzlich zusammen mit der Gruppe $-(-C_nH_{2n}-)-$ einen $C_5$–$C_{12}$ –Cycloalkylrest bilden kann,

n die Zahlen 1 bis 20,

m 1 oder 2, wobei jedoch m nicht 2 ist, wenn $R_1$ für Wasserstoff oder $C_1$–$C_4$ Alkyl steht,

A eine direkte C–C-Bindung, wenn m = 1 ist, oder ein Rest $-\overset{|}{C}H-$, wenn m = 2 ist,

Y –O– oder $-N(R_5)-$, worin $R_5$ Wasserstoff, $C_1$–$C_{18}$ Alkyl, $C_3$–$C_{12}$ Alkenyl, $C_3$–$C_{12}$ Cycloalkyl, Phenyl, $C_7$–$C_{14}$ Aralkyl, $C_7$–$C_{14}$ Alkaryl, $C_2$–$C_{11}$ Alkoxyalkyl oder eine Gruppe der Formel IV

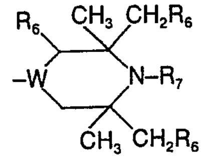 (IV)

ist, bedeutet

M eine direkte Bindung, –O–, –S–, –S–S–, –SO–, $-SO_2-$ oder eine Gruppe $-CH_2OCH_2-$, $-CH_2SCH_2-$, $-CH(R_8)-$ oder $-N(R_9)-$ ist, worin $R_8$ Wasserstoff, $C_1-C_{12}$ Alkyl oder durch 1–3 Schwefelatome unterbrochenes $C_3-C_8$ Alkyl und $R_9$ Wasserstoff, $C_1-C_{18}$ Alkyl, unsubstituiertes oder durch $C_1-C_4$ Alkyl substituiertes Phenyl oder Benzyl bedeuten,

X eine Gruppe der Formel

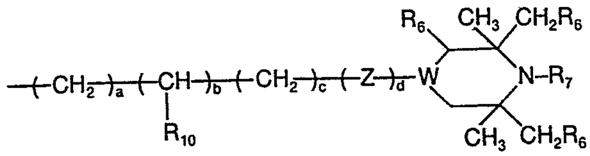

oder der Formel

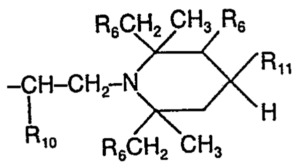

ist,

a die Zahlen 0 bis 10 bedeutet,

b, c und d unabhängig voneinander die Zahlen 0 oder 1 bedeuten, wobei, wenn d = 1 ist, die Summe a + b + c ≠ 0 sein muss,

$R_6$ Wasserstoff oder Methyl,

$R_7$ Hydroxy, $C_1-C_{12}$ Alkyl, $C_3-C_6$ Alkenylmethyl, $C_3-C_4$ Alkinylmethyl, $C_7-C_{14}$ Aralkyl, Glycidyl, durch Halogen, Cyano, $-COOR_{12}$ oder $-CON(R_{13})(R_{14})$ substituiertes $C_1-C_4$ Alkyl, eine Gruppe $-COR_{15}$, $-COOR_{12}$, $-CON(R_{13})(R_{14})$, $-CH_2-CH(R_{16})-OR_{17}$, $-SOR_{18}$, $-SO_2R_{18}$, $-OR_{12}$, $-OCOR_{15}$ ist, wobei $R_{12}$ $C_1-C_{12}$ Alkyl, Allyl, Cyclohexyl oder Benzyl, $R_{13}$ $C_1-C_{12}$ Alkyl, Allyl, Cyclohexyl, Benzyl, Phenyl oder $C_{7-10}$ Alkylphenyl, $R_{14}$ Wasserstoff $C_1-C_{12}$ Alkyl, Allyl, Cyclohexyl oder Benzyl sind oder $R_{13}$ und $R_{14}$ zusammen mit dem N-Atom an das sie gebunden sind einen 5- oder 6-gliedrigen heterocyclischen Ring bilden, $R_{15}$ Wasserstoff, $C_1-C_{12}$ Alkyl, $C_2-C_6$ Alkenyl, Chloromethyl, $C_5-C_8$ Cycloalkyl, $C_7-C_{14}$ Aralkyl, Phenyl, $C_7-C_{10}$ Alkylphenyl oder durch 1 oder 2 $C_1-C_4$ Alkyl und 1 Hydroxy substituiertes Phenyl, Phenylmethyl oder Phenylethyl, $R_{16}$ Wasserstoff, $C_1-C_4$ Alkyl, $C_3-C_4$ Alkoxyalkyl, Phenyl oder Phenoxymethyl, $R_{17}$ Wasserstoff, $C_1-C_{12}$ Alkyl, eine Gruppe $-COR_{15}$, $-CON(R_{13})(R_{14})$ oder eine Gruppe der Formel

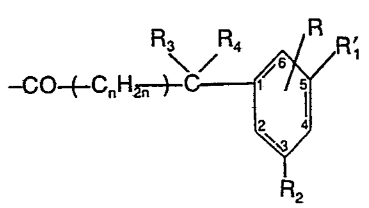

worin $R_1'$ Wasserstoff oder $C_1-C_4$ Alkyl ist und $R_{18}$ $C_1-C_{12}$ Alkyl, Phenyl oder $C_7-C_{10}$ Alkylphenyl bedeuten, oder $R_7$ eine Gruppe der Formel

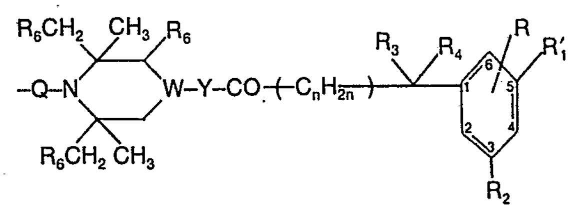

ist, worin

Q eine Gruppe $-(-C_rH_{2r}-)-$, worin r die Zahlen 2 bis 12 bedeutet, ist, oder Q ist $C_4-C_8$ Alkenylen, $C_5-C_{12}$ Cycloalkylen, Phenylen, Xylylen, Bitolylen oder eine Gruppe $-CO-(-C_rH_{2r}-)-CO-$, –O– oder $-N(R_{19})-$ ist, worin $R_{19}$ Wasserstoff, $C_1-C_{18}$ Alkyl, $C_3-C_{12}$ Alkenyl, $C_3-C_{12}$ Cycloalkyl, Phenyl, $C_7-C_{14}$ Alkaryl, $C_7-C_{14}$ Aralkyl, $C_2-C_{11}$ Alkoxyalkyl, eine Gruppe $-COR_{20}$, $-COOR_{21}$, $-CON(R_{22})(R_{23})$, $-CH_2-CH(R_{24})-OR_{25}$, $-SOR_{26}$ oder $-SO_2R_{26}$ ist, worin $R_{20}$ eine der für $R_{15}$ angegebenen Bedeutungen oder ein heterozyklischer Ring ist, $R_{21}$ eine der für $R_{12}$ angegebenen Bedeutungen hat, $R_{22}$ eine der für $R_{13}$ angegebenen Bedeutungen hat, $R_{23}$ eine der für $R_{14}$ angegebenen Bedeutungen hat, $R_{24}$ eine der für $R_{16}$ angegebenen Bedeutungen hat, $R_{25}$ eine der für $R_{17}$ angegebenen Bedeutungen hat und $R_{26}$ eine der für $R_{18}$ angegebenen Bedeutungen hat, oder $R_{19}$ eine Gruppe der Formel IV ist,

$R_{10}$ Wasserstoff, $C_1-C_{18}$ Alkyl, $C_7-C_{23}$ Phenoxyalkyl, Phenyl, $C_7-C_{14}$ Aralkyl, $C_2-C_{11}$ Alkoxyalkyl oder eine Gruppe

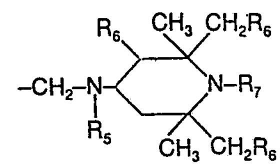

ist,

$R_{11}$ Wasserstoff, $-OR_{27}$, $-OCOR_{28}$, $-N(R_{29})-COR_{28}$, $-OSO_2R_{28}$, $-N(R_{29})-SO_2R_{28}$ ist, wobei $R_{27}$ Wasserstoff, $C_1-C_{12}$ Alkyl, Allyl, Benzyl ist, $R_{28}$ Wasserstoff, $C_1-C_{12}$ Alkyl, $C_2-C_6$ Alkenyl, Chlormethyl, $C_5-C_8$ Cycloalkyl, $C_7-C_9$ Phenylalkyl, $C_7-C_{10}$ Alkylphenyl, Phenyl oder eine Gruppe der Formel

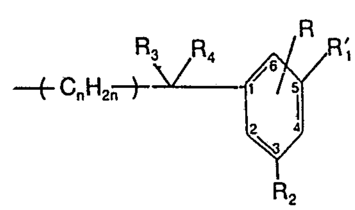

bedeutet und $R_{29}$ Wasserstoff, $C_1-C_{12}$ Alkyl, $C_5-C_8$ Cycloalkyl oder Benzyl ist und

W eine der Gruppen

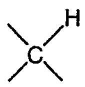

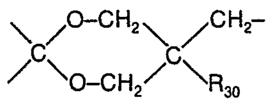

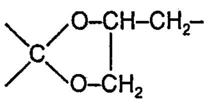

oder

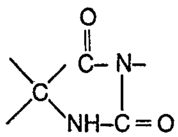

ist, worin $R_{30}$ Methyl oder Ethyl bedeutet mit der Bedingung, dass wenn W eine cyclische Ketalstruktur bedeutet, d = 0 ist und die Summe a + b + c auch 0 sein muss und, wenn W eine Hydantoinstruktur bedeutet, d = 0 und die Summe a + b + c ≠ 0 sein müssen,

wobei die wiederholt erwähnten Reste und Symbole immer die erstmals angegebene Bedeutung haben und bei wiederholtem Vorkommen der Gruppe

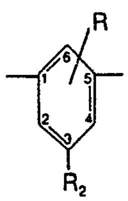

sich R immer in derselben Stellung befindet.

2. Farbphotographisches Aufzeichnungsmaterial gemäss Anspruch 1, dadurch gekennzeichnet, dass sich in den Verbindungen der Formel I der Rest R jeweils in 6-Stellung befindet.

3. Farbphotographisches Aufzeichnungsmaterial gemäss Anspruch 1, dadurch gekennzeichnet, dass sich in den Verbindungen der Formel I der Rest R jeweils in 4-Stellung befindet.

4. Farbphotographisches Aufzeichnungsmaterial gemäss Anspruch 1, dadurch gekennzeichnet, dass sich in den Verbindungen der Formel I der Rest R jeweils in 2-Stellung befindet.

5. Farbphotographisches Aufzeichnungsmaterial gemäss Anspruch 1, dadurch gekennzeichnet, dass es als Stabilisator mindestens eine Polyalkylpiperidinverbindung der Formel Ia enthält

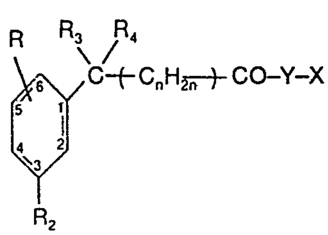

(Ia).

6. Farbphotographisches Aufzeichnungsmaterial gemäss Anspruch 1, dadurch gekennzeichnet, dass es als Stabilisator mindestens eine Polyalkylpiperidinverbindung der Formel V

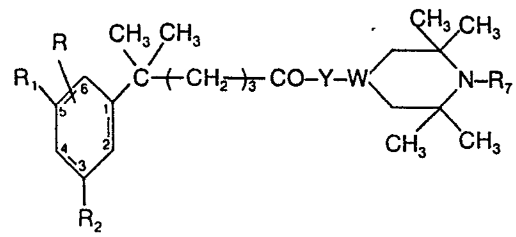

(V)

enthält, worin

R eine OH-Gruppe ist, die sich in 2-, 4- oder 6-Stellung befindet,

$R_1$ Wasserstoff, $C_1$–$C_4$ Alkyl, eine Gruppe der Formel VI

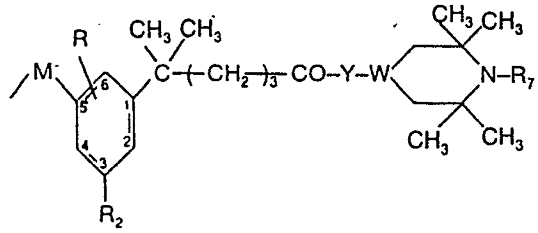

(VI)

oder eine Gruppe der Formel VII

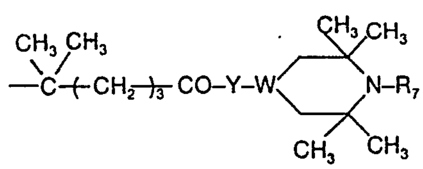

(VII)

ist,

$R_2$ $C_1$–$C_4$ Alkyl oder eine Gruppe der Formel VII bedeutet

Y –O– oder –N($R_5$)–, worin $R_5$ Wasserstoff, $C_1$–$C_{12}$ Alkyl, Cyclohexyl, $C_3$–$C_4$ Alkoxyalkyl oder eine Gruppe der Formel VIII

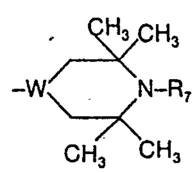

(VIII)

ist, bedeutet,

M –CH($R_8$)– oder –S– ist, wobei $R_8$ Wasserstoff oder $C_1$–$C_4$ Alkyl bedeutet,

$R_7$ Hydroxy, Methyl, Allyl, Benzyl, 2-Hydroxyethyl, Acetyl, Acroyl oder eine Gruppe –CON($R_{13}$)($R_{14}$), worin $R_{13}$ $C_1$–$C_8$ Alkyl, Cyclohexyl oder Phenyl und $R_{14}$ Wasserstoff, $C_1$–$C_8$ Alkyl oder Cyclohexyl bedeuten, ist, oder

$R_7$ eine Gruppe der Formel

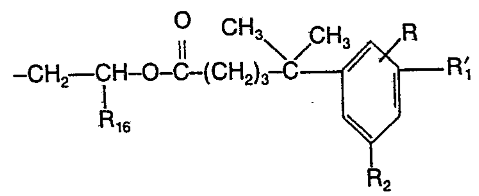

ist, worin $R_{16}$ Wasserstoff oder Methyl bedeutet und

W eine der Gruppen

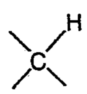

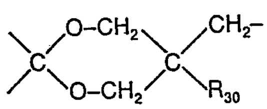

oder

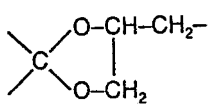

ist, worin $R_{30}$ Methyl oder Ethyl bedeutet, wobei die in diesem Anspruch wiederholt erwähnten Reste immer die in diesem Anspruch erstmals angegebene Bedeutung haben und bei wiederholtem Vorkommen der Gruppe

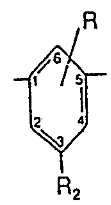

sich R immer in derselben Stellung befindet.

7. Farbphotographisches Aufzeichnungsmaterial gemäss Anspruch 6, dadurch gekennzeichnet, dass sich in den Verbindungen der Formel V der Rest R jeweils in 6-Stellung befindet.

8. Farbphotographisches Aufzeichnungsmaterial gemäss Anspruch 6, dadurch gekennzeichnet, dass sich in den Verbindungen der Formel V der Rest R jeweils in 4-Stellung befindet.

9. Farbphotographisches Aufzeichnungsmaterial gemäss Anspruch 6, dadurch gekennzeichnet, dass sich in den Verbindungen der Formel V der Rest R jeweils in 2-Stellung befindet.

10. Farbphotographisches Aufzeichnungsmaterial gemäss Anspruch 1, dadurch gekennzeichnet, dass es als Stabilisator mindestens eine Polyalkylpiperidinverbindung der Formel IX

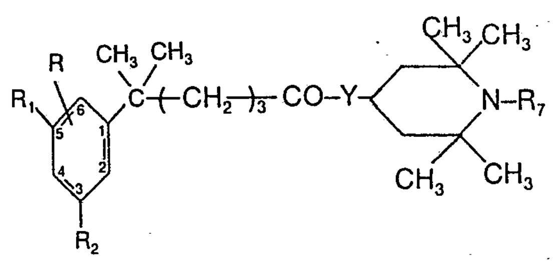

(IX)

enthält, worin

R eine OH-Gruppe ist, die sich in 2-, 4- oder 6-Stellung befindet

$R_1$ Wasserstoff, $C_1$–$C_4$ Alkyl oder eine Gruppe der Formel X

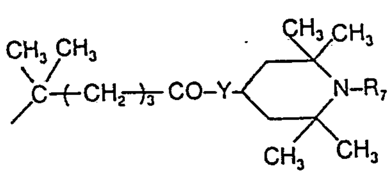

(X)

$R_2$ $C_1$–$C_4$ Alkyl oder eine Gruppe der Formel X

Y –O– oder –$N(R_5)$–, worin $R_5$ Wasserstoff oder $C_1$–$C_8$ Alkyl bedeutet und

$R_7$ Hydroxy, Methyl, Allyl, Benzyl, 2-Hydroxyethyl, Acetyl, Acryloyl oder eine Gruppe der Formel

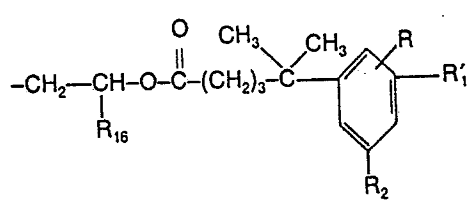

ist, worin $R_{16}$ Wasserstoff oder Methyl bedeutet.

11. Farbphotographisches Aufzeichnungsmaterial gemäss Anspruch 10, dadurch gekennzeichnet, dass sich in den Verbindungen der Formel IX der Rest R jeweils in 6-Stellung befindet.

12. Farbphotographisches Aufzeichnungsmaterial gemäss Anspruch 10, dadurch gekennzeichnet, dass sich in den Verbindungen der Formel IX der Rest R jeweils in 4-Stellung befindet.

13. Farbphotographisches Aufzeichnungsmaterial gemäss Anspruch 10, dadurch gekennzeichnet,

net, dass sich in den Verbindungen der Formel IX der Rest R jeweils in 2-Stellung befindet.

14. Farbphotographisches Aufzeichnungsmaterial gemäss Anspruch 1, dadurch gekennzeichnet, dass es als Stabilisator eine Polyalkylpiperidinverbindung der Formel XVI enthält

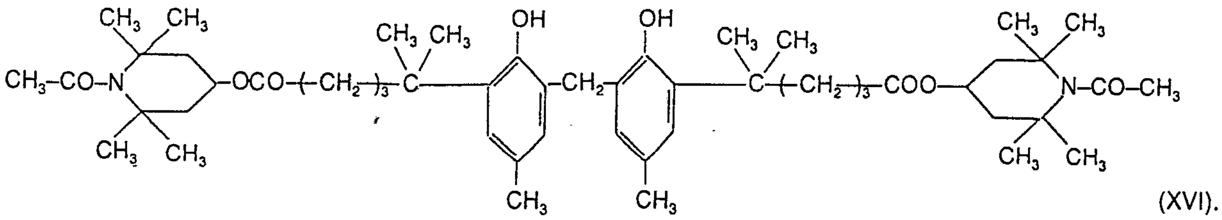

(XVI).

15. Farbphotographisches Aufzeichnungsmaterial gemäss Anspruch 1, dadurch gekennzeichnet, dass es als Stabilisator eine Polyalkylpiperidinverbindung der Formel XVII enthält

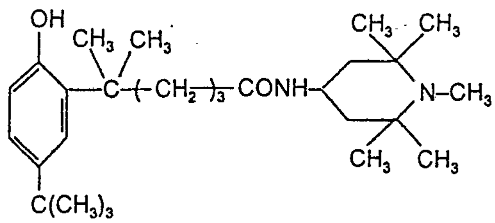

(XVII).

16. Farbphotographisches Aufzeichnungsmaterial gemäss Anspruch 1, dadurch gekennzeichnet, dass es als Stabilisator eine Polyalkylpiperidinverbindung der Formel XVIII enthält

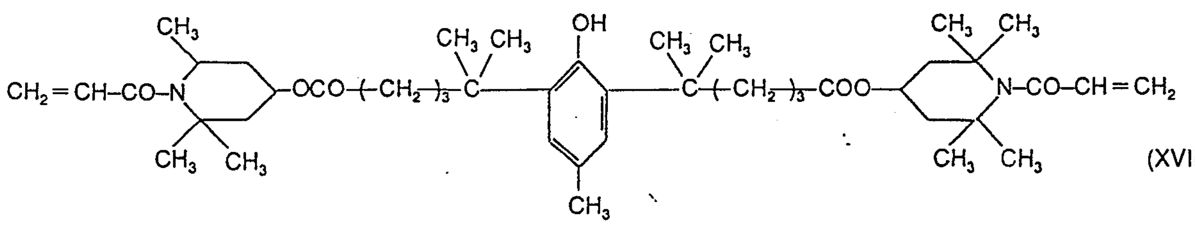

(XVIII).

17. Farbphotographisches Aufzeichnungsmaterial gemäss Anspruch 1, dadurch gekennzeichnet, dass es als Stabilisator eine Polyalkylpiperidinverbindung der Formel XIX enthält

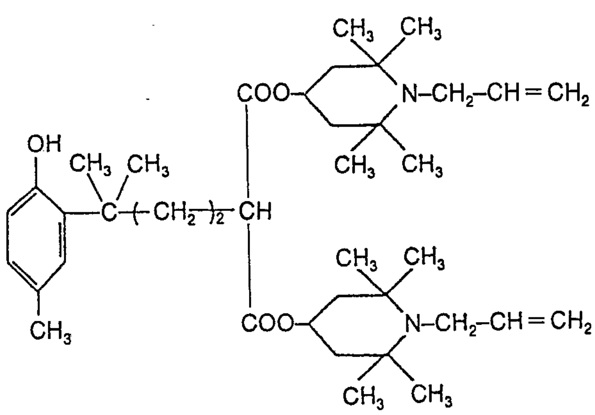

(XIX).

18. Farbphotographisches Aufzeichnungsmaterial gemäss Anspruch 1, dadurch gekennzeichnet, dass es als Stabilisator eine Polypiperidinverbindung der Formel XX enthält

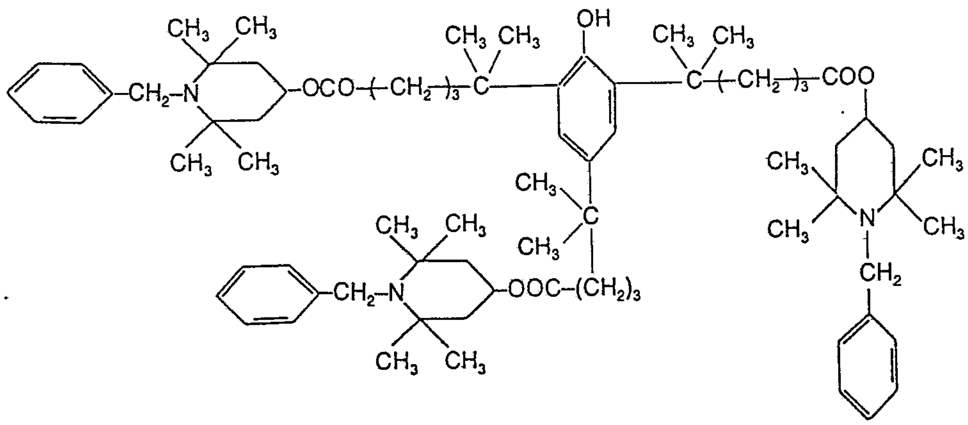

(XX).

19. Farbphotographisches Aufzeichnungsmaterial gemäss Anspruch 1, dadurch gekennzeichnet, dass es die Stabilisatoren der Formel I in Kombination mit Blaugrün-, Purpur- und Gelbkupplern enthält.

20. Farbphotographisches Aufzeichnungsmaterial gemäss Anspruch 1, dadurch gekennzeichnet, dass es die Stabilisatoren der Formel I in Kombination mit Ultraviolettabsorbern enthält.

21. Farbphotographisches Aufzeichnungsmaterial gemäss Anspruch 2, dadurch gekennzeichnet, dass die Ultraviolettabsorber Verbindungen von Benzophenon-, Acrylnitril-, Thiazolidon-, Benztriazol-, Oxazol-, Thiazol- oder Imidazoltyp sind.

22. Farbphotographisches Aufzeichnungsmaterial gemäss Anspruch 1, dadurch gekennzeichnet, dass es die Stabilisatoren der Formel I in Kombination mit Blaugrün-, Purpur- und Gelbkupplern und mit UV-Absorbern in der gleichen Schicht enthält.

23. Farbphotographisches Aufzeichnungsmaterial gemäss Anspruch 1, dadurch gekennzeichnet, dass es 1 bis 2000 mg des Stabilisators der Formel I pro $m^2$ der Schicht enthält, in die es eingearbeitet ist.

24. Verfahren zur Herstellung von photographischen Farbbildern, durch bildmässige Belichtung und Farbentwicklung eines farbphotographischen Aufzeichnungsmaterials gemäss Anspruch 1.

## Claims

1. A colour-photographic recording material which, in at least one light-sensitive silver halide emulsion layer, an interlayer and/or a protective layer, contains at least one polyalkylpiperidine compound as a stabiliser, wherein the polyalkylpiperidine compound is of the formula I

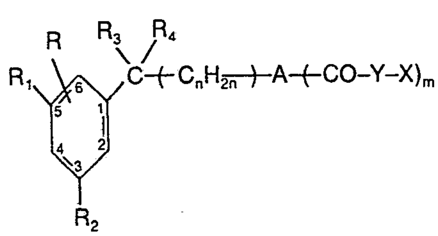

(I)

in which R is an OH group in the 2-position, 4-position or 6-position, $R_1$ is hydrogen, $C_1$–$C_4$-alkyl, a group of the formula II

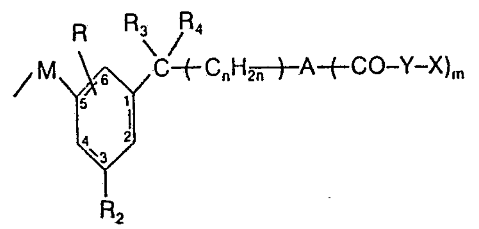

(II)

or a group of the formula III

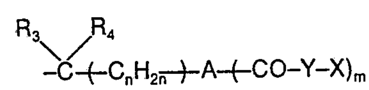

(III)

$R_2$ is $C_1$–$C_4$-alkyl, a group of the formula III or a group of the formula –CO–Y–X, $R_3$ and $R_4$ independently of one another are $C_1$–$C_8$-alkyl and, in addition, $R_4$ can, together with the group $-(C_nH_{2n})-$, form a $C_5$–$C_{12}$-cycloalkyl radical, n is a number from 1 to 20, m is 1 or 2, but m is not 2 if $R_1$ is hydrogen or $C_1$–$C_4$-alkyl, A is a direct C–C bond if m = 1 or a radical –CH– if m = 2, Y is –O– or $-N(R_5)-$, wherein $R_5$ is hydrogen, $C_1$–$C_{18}$-alkyl, $C_3$–$C_{12}$-alkenyl, $C_3$–$C_{12}$-cycloalkyl, phenyl, $C_7$–$C_{14}$-aralkyl, $C_7$–$C_{14}$-alkaryl, $C_2$–$C_{11}$-alkoxyalkyl or a group of the formula IV

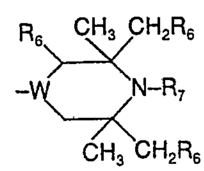

(IV)

M is a direct bond, –O–, –S–, –S–S–, –SO–, $-SO_2-$ or a group $-CH_2OCH_2-$, $-CH_2SCH_2-$, $-CH(R_8)-$ or $-N(R_9)-$, wherein $R_8$ is hydrogen, $C_1$–$C_{12}$-alkyl or $C_3$–$C_8$-alkyl interrupted by 1–3 sulfur atoms, and $R_9$ is hydrogen, $C_1$–$C_{18}$-alkyl or unsubstituted or $C_1$–$C_4$-alkyl-substituted phenyl or benzyl, X is a group of the formula

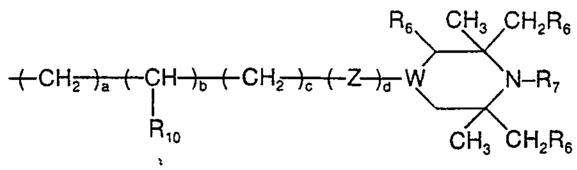

or of the formula

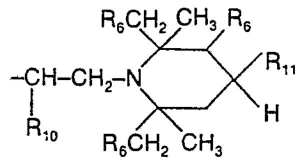

a is one of the numbers from 0 to 10, b, c and d independently of one another are the number 0 or 1, it being necessary for the sum a + b + c$\neq$ 0 if d = 1, $R_6$ is hydrogen or methyl, $R_7$ is hydroxyl, $C_1$–$C_{12}$-alkyl, $C_3$–$C_6$-alkenylmethyl, $C_3$–$C_4$-alkynylmethyl, $C_7$–$C_{14}$-aralkyl, glycidyl, $C_1$–$C_4$-alkyl, substituted by halogen, cyano, $-COOR_{12}$ or $-CON(R_{13})-(R_{14})$, a group $-COR_{15}$, $-COOR_{12}$, $-CON(R_{13})(R_{14})$, $-CH_2-CH(R_{16})-OR_{17}$, $-SOR_{18}$, $-SO_2R_{18}$, $-OR_{12}$ or $-OCOR_{15}$, $R_{12}$ being $C_1$–$C_{12}$-alkyl, allyl, cyclohexyl or benzyl, $R_{13}$ being $C_1$–$C_{12}$-alkyl, allyl, cyclohexyl, benzyl, phenyl or $C_7$–$C_{10}$-alkylphenyl, $R_{14}$ being hydrogen, $C_1$–$C_{12}$-alkyl, allyl, cyclohexyl or benzyl, or $R_{13}$ and $R_{14}$, together with the N atom to which they are attached, forming a 5-membered or 6-membered heterocyclic ring, $R_{15}$ being hydrogen, $C_1$–$C_{12}$-alkyl, $C_2$–$C_6$-alkenyl, chloromethyl, $C_5$–$C_8$-cycloalkyl, $C_7$–$C_{14}$-aralkyl, phenyl, $C_7$–$C_{10}$-alkylphenyl or phenyl, phenylmethyl or phenylethyl which are substituted by 1 or 2 $C_1$–$C_4$-alkyls and 1 hydroxyl, $R_{16}$ being hydrogen, $C_1$–$C_4$-alkyl, $C_3$–$C_4$-alkoxyalkyl, phenyl or phenoxymethyl, $R_{17}$ being hydrogen, $C_1$–$C_{12}$-alkyl, a group $-COR_{15}$ or $-CON(R_{13})(R_{14})$ or a group of the formula

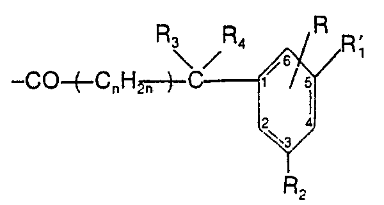

in which $R_1'$ is hydrogen or $C_1$–$C_4$-alkyl, and $R_{18}$ being $C_1$–$C_{12}$-alkyl, phenyl or $C_7$–$C_{10}$-alkylphenyl, or $R_7$ is a group of the formula

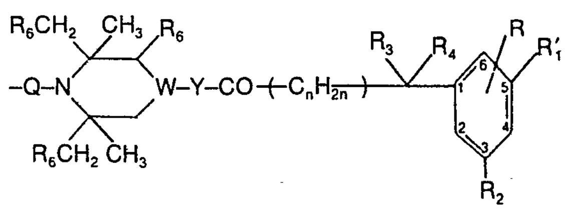

in which Q is a group $-(C_rH_{2r})-$ wherein r is one of the numbers 2 to 12, or Q is $C_4$–$C_8$-alkenylene, $C_5$–$C_{12}$-cycloalkylene, phenylene, xylylene, bitolylene or a group $-CO-(C_rH_{2r})-CO-$, Z is $-O-$ or $-N(R_{19})-$ wherein $R_{19}$ is hydrogen, $C_1$–$C_{18}$-alkyl, $C_3$–$C_{12}$-alkenyl, $C_3$–$C_{12}$-cycloalkyl, phenyl, $C_7$–$C_{14}$-alkaryl, $C_7$–$C_{14}$-aralkyl, $C_2$–$C_{11}$-alkoxyalkyl, a group $-COR_{20}$, $-COOR_{21}$, $-CON(R_{22})(R_{23})$, $-CH_2-CH(R_{24})-OR_{25}$, $-SOR_{26}$ or $-SO_2R_{26}$, $R_{20}$ having one of the meanings defined for $R_{15}$ or being a heterocyclic ring, $R_{21}$ having one of the meanings defined for $R_{12}$, $R_{22}$ having one of the meanings defined for $R_{13}$, $R_{23}$ having one of the meanings defined for $R_{14}$, $R_{24}$ having one of the meanings defined for $R_{16}$, $R_{25}$ having one of the meanings defined for $R_{17}$ and $R_{26}$ having one of the meanings defined for $R_{18}$, or $R_{19}$ is a group of the formula IV, $R_{10}$ is hydrogen, $C_1$–$C_{18}$-alkyl, $C_7$–$C_{23}$-phenoxyalkyl, phenyl, $C_7$–$C_{14}$-aralkyl, $C_2$–$C_{11}$-alkoxyalkyl or a group

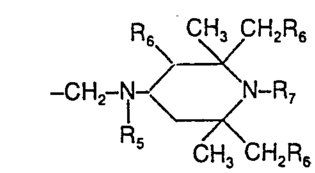

$R_{11}$ is hydrogen, $-OR_{27}$, $-OCOR_{28}$, $-N(R_{29})-COR_{28}$, $-OSO_2R_{28}$ or $-N(R_{29})-SO_2R_{28}$, $R_{27}$ being hydrogen, $C_1$–$C_{12}$-alkyl, allyl or benzyl, $R_{28}$ being hydrogen, $C_1$–$C_{12}$-alkyl, $C_2$–$C_6$-alkenyl, chloromethyl, $C_5$–$C_8$-cycloalkyl, $C_7$–$C_9$-phenylalkyl, $C_7$–$C_{10}$-alkylphenyl, phenyl or a group of the formula

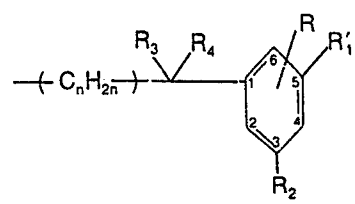

and $R_{29}$ being hydrogen, $C_1$–$C_{12}$-alkyl, $C_5$–$C_8$-cycloalkyl or benzyl, and W being one of the groups

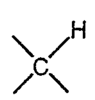

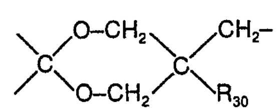

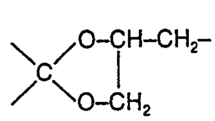

or

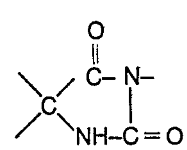

in which $R_{30}$ is methyl or ethyl, with the proviso that, if W is a cyclic ketal structure, d is 0 and the sum a + b + c must also be 0 and, if W is a hydantoin structure, d must be 0 and the sum a + b + c must be $\neq$ 0, the repeatedly mentioned radicals and symbols always being as defined in the first instance and, with repeated occurrence of the group

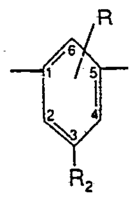

R always being in the same position.

2. A colour-photographic recording material according to Claim 1, wherein each radical R in the compounds of the formula I is in the 6-position.

3. A colour-photographic recording material according to Claim 1, wherein each radical R in the compounds of the formula I is in the 4-position.

4. A colour-photographic recording material according to Claim 1, wherein each radical R in the compounds of the formula I is in the 2-position.

5. A colour-photographic recording material according to Claim 1, which contains, as the stabiliser, at least one polyalkylpiperidine compound of the formula Ia

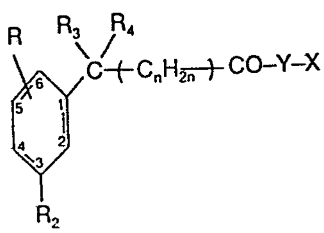

(Ia) .

6. A colour-photographic recording material according to Claim 1, which contains, as the stabiliser, at least one polyalkylpiperidine compound of the formula V

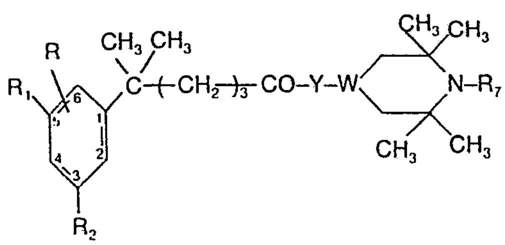

in which R is an OH group in the 2-position, 4-position or 6-position, $R_1$ is hydrogen, $C_1$–$C_4$-alkyl, a group of the formula VI

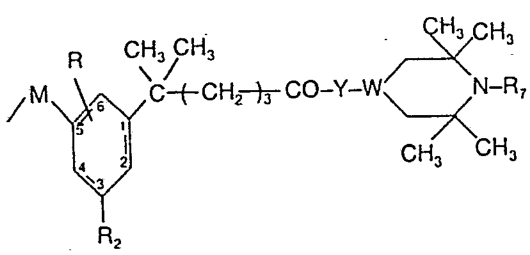

(VI)

or a group of the formula VII

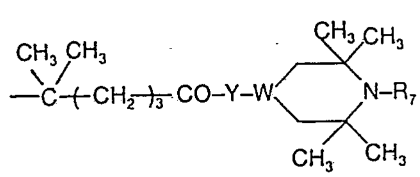

(VII)

$R_2$ is $C_1$–$C_4$-alkyl or a group of the formula VII, Y is –O– or –N($R_5$)–, being hydrogen, $C_1$–$C_{12}$-alkyl, cyclohexyl, $C_3$–$C_4$-alkoxyalkyl or a group of the formula VIII

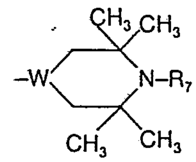

(VIII)

M is –CH($R_8$)– or –S–, $R_8$ being hydrogen or $C_1$–$C_4$-alkyl, $R_7$ is hydroxyl, methyl, allyl, benzyl, 2-hydroxyethyl, acetyl, acroyl or a group –CON($R_{13}$)($R_{14}$), wherein $R_{13}$ is $C_1$–$C_8$-alkyl, cyclohexyl or phenyl and $R_{14}$ is hydrogen, $C_1$–$C_8$-alkyl or cyclohexyl, or $R_7$ is a group of the formula

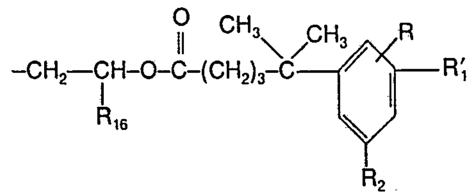

in which $R_{16}$ is hydrogen or methyl, and W is one of the groups

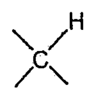

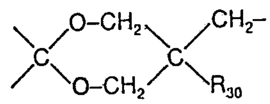

or

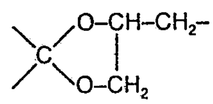

in which $R_{30}$ is methyl of ethyl, the radicals repeatedly mentioned in this claim always being as defined in this claim in the first instance and, with repeated occurrence of the group

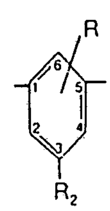

R always being in the same position.

7. A colour-photographic recording material according to Claim 6, wherein each radical R in the compounds of the formula V is in the 6-position.

8. A colour-photographic recording material according to Claim 6, wherein each radical R in the compounds of the formula V is in the 4-position.

9. A colour-photographic recording material according to Claim 6, wherein each radical R in the compounds of the formula V is in the 2-position.

10. A colour-photographic recording material according to Claim 1, which contains, as the stabiliser, at least one polyalkylpiperidine compound of the formula IX

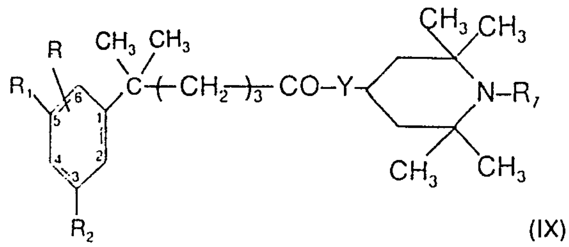

(IX)

in which R is an OH group in the 2-position, 4-position or 6-position, $R_1$ is hydrogen, $C_1$–$C_4$-alkyl or a group of the formula X

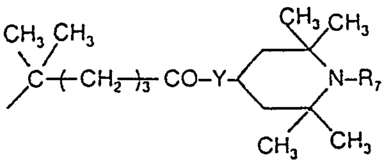

(X)

$R_2$ is $C_1$–$C_4$-alkyl or a group of the formula X, Y is –O– or $-N(R_5)-$, wherein $R_5$ is hydrogen or $C_1$–$C_8$-alkyl, and $R_7$ is hydroxyl, methyl, allyl, benzyl, 2-hydroxyethyl, acetyl, acryloyl or a group of the formula

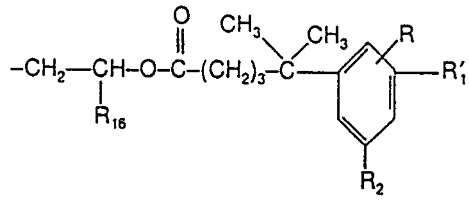

in which $R_{16}$ is hydrogen or methyl.

11. A colour-photographic recording material according to Claim 10, wherein each radical R in the compounds of the formula IX is in the 6-position.

12. A colour-photographic recording material according to Claim 10, wherein each radical R in the compounds of the formula IX is in the 4-position.

13. A colour-photographic recording material according to Claim 10, wherein each radical R in the compounds of the formula IX is in the 2-position.

14. A colour-photographic recording material according to Claim 1, which contains, as the stabiliser, a polyalkylpiperidine compound of the formula XVI

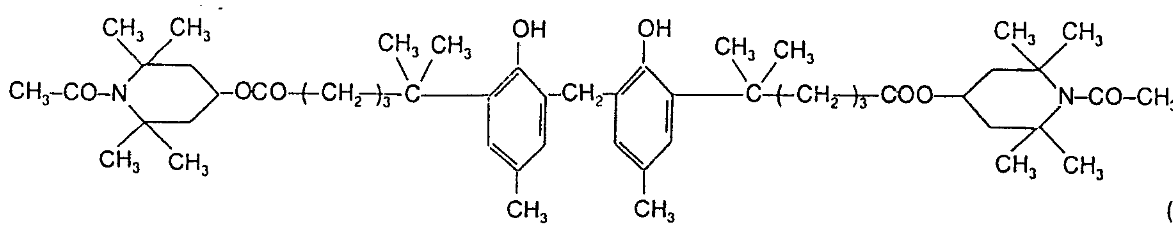

(XVI).

15. A colour-photographic recording material according to Claim 1, which contains, as the stabiliser, a polyalkylpiperidine compound of the formula XVII

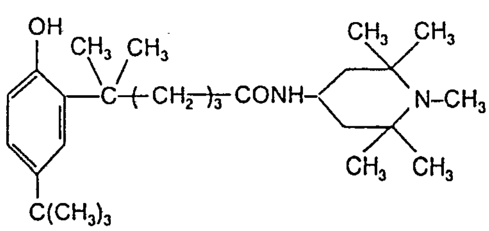

(XVII).

16. A colour-photographic recording material according to Claim 1, which contains, as the stabiliser, a polyalkylpiperidine compound of the formula XVIII

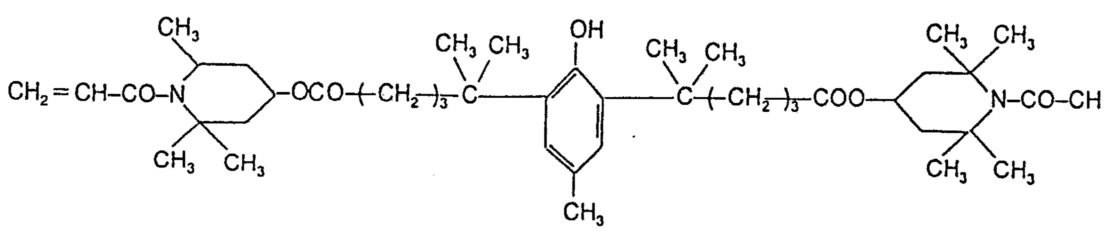

(XVIII).

17. A colour-photographic recording material according to Claim 1, which contains, as the stabiliser, a polyalkylpiperidine compound of the formula XIX

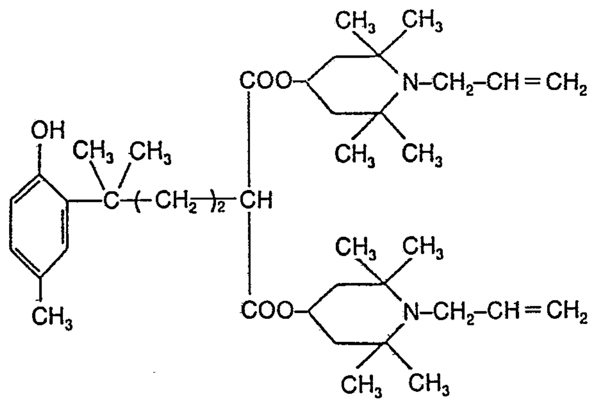

(XIX).

18. A colour-photographic recording material according to Claim 1, which contains, as the stabiliser, a polyalkylpiperidine compound of the formula XX

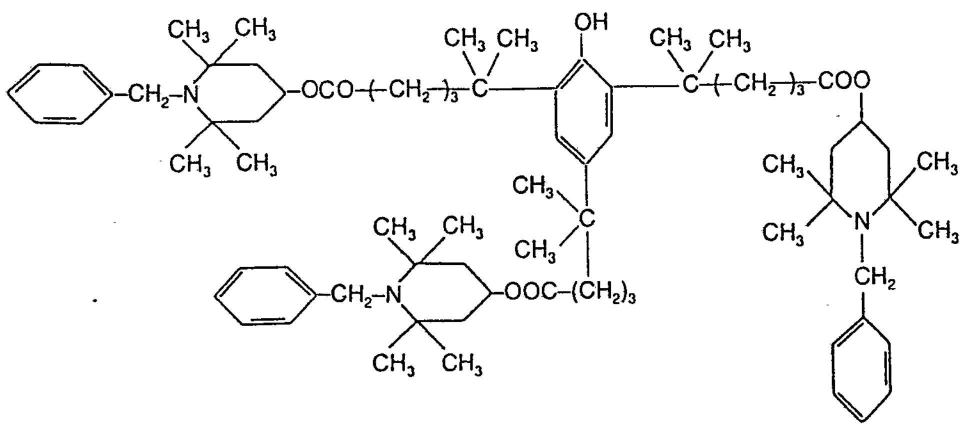

(XX).

19. A colour-photographic recording material according to Claim 1, which contains a stabiliser of the formula I in combination with cyan, magenta and yellow couplers.

20. A colour-photographic recording material according to Claim 1, which contains a stabiliser of the formula I in combination with ultraviolet absorber.

21. A colour-photographic recording material according to Claim 20, wherein the ultraviolet absorber is a compound of the benzophenone, acrylonitrile, thiazolidone, benzotriazole, oxazole, thiazole or imidazol types.

22. A colour-photographic recording material according to Claim 1, which contains the stabiliser of the formula I in combination with cyan, magenta and yellow couplers and with ultraviolet absorbers in the same layer.

23. A colour-photographic recording material according to Claim 1, which contains 1 to 2,000 mg of the stabiliser of the formula I per $m^2$ of the layer into which it is incorporated.

24. A process for the production of photographic colour images by imagewise exposure and colour development of a colour-photographic recording material according to Claim 1.

**Revendications**

1. Matière pour enregistrement photographique en couleurs qui contient, comme stabilisant, au moins un composé polyalkyl-pipéridinique dans au moins une couche d'émulsion d'halogénure d'argent photosensible, une couche intermédiaire et/ou une couche de protection, matière caractérisée en ce que le composé polyalkyl-pipéridinique répond à la formule I:

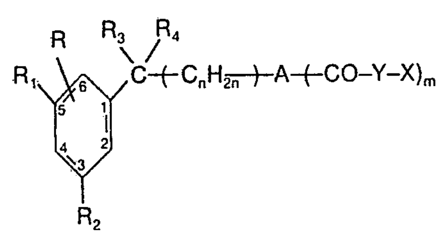

(I)

dans laquelle:

R représente un radical –OH qui se trouve en position 2, 4 ou 6,

$R_1$ représente l'hydrogène, un alkyle en $C_1$–$C_4$, un radical de formule II:

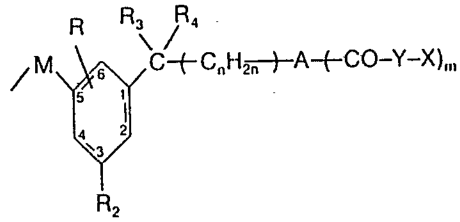

(II)

ou un radical de formule III:

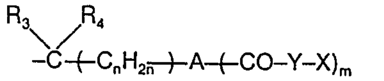
(III)

$R_2$ représente un alkyle en $C_1$–$C_4$, un radical de formule III ou un radical de formule –CO–Y–X,

$R_3$ et $R_4$ représentent chacun, indépendamment l'un de l'autre, un alkyle en $C_1$–$C_8$, $R_4$ pouvant en outre former un radical cycloalkyle en $C_5$–$C_{12}$ avec le radical $-(C_nH_{2n}-)-$,

n désigne un nombre de 1 à 20,

m est égal à 1 ou à 2, mais, lorsque $R_1$ représente l'hydrogène ou un alkyle en $C_1$–$C_4$, m n'est pas égal à 2,

A représente une liaison directe C–C lorsque m est égal à 1 ou représente un radical –CH– lorsque m est égal à 2,

Y représente –O–, ou un radical $-N(R_5)-$ dans lequel $R_5$ représente l'hydrogène, un alkyle en $C_1$–$C_{18}$, un alcényle en $C_3$–$C_{12}$, un cycloalkyle en $C_3$–$C_{12}$, un phényle, un aralkyle en $C_7$–$C_{14}$, un alkylaryle en $C_7$–$C_{14}$, un alcoxyalkyle en $C_2$–$C_{11}$ ou un radical de formule IV:

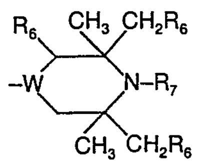
(IV)

M représente une liaison directe, un radical –O–, –S–, –S–S–, –SO–, $-SO_2-$, $-CH_2OCH_2-$ ou $-CH_2SCH_2-$, ou l'un des radicaux $-CH(R_8)-$ et $-N(R_9)-$ dans lesquels $R_8$ représente l'hydrogène, un alkyle en $C_1$–$C_{12}$ ou un alkyle en $C_3$–$C_8$ interrompu par 1 à 3 atomes de soufre et $R_9$ représente l'hydrogène, un alkyle en $C_1$–$C_{18}$, ou un radical benzyle ou phényle non substitué ou porteur d'un alkyle en $C_1$–$C_4$,

X représente un radical de formule;

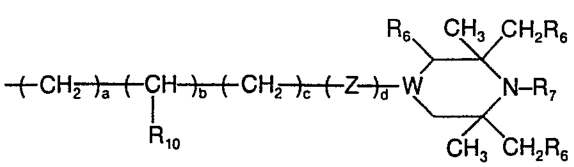

ou un radical de formule:

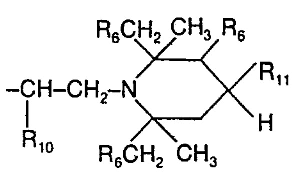
,

a représente un nombre de 0 à 10,

b, c et d représentent chacun, indépendamment les uns des autres, le nombre 0 ou le nombre 1 avec la condition que, lorsque d est égal à 1, la somme (a + b + c) soit différente de zéro,

$R_6$ représente l'hydrogène ou un méthyle,

$R_7$ représente un hydroxy, un alkyle en $C_{11}$–$C_{12}$, un alcénylméthyle en $C_3$–$C_6$, un alcynylméthyle en $C_3$ ou $C_4$, un aralkyle en $C_7$–$C_{14}$, un glycidyle, un alkyle en $C_1$–$C_4$ porteur d'un halogène, d'un cyano, d'un radical de formule $-COOR_{12}$ ou de formule $-CON(R_{13})(R_{14})$, ou un radical répondant à l'une des formules $-COR_{15}$, $-COOR_{12}$, $-CON(R_{13})(R_{14})$, $-CH_2-CH(R_{16})-OR_{17}$, $-SOR_{18}$, $-SO_2R_{18}$, $-OR_{12}$ et $-OCOR_{15}$, dans lesquelles $R_{12}$ représente un alkyle en $C_1$–$C_{12}$, un allyle, un cyclohexyle ou un benzyle, $R_{13}$ un alkyle en $C_1$–$C_{12}$, un allyle, un cyclohexyle, un benzyle, un phényle ou un alkylphényle en $C_7$–$C_{10}$, $R_{14}$ l'hydrogène, un alkyle en $C_1$–$C_{12}$, un allyle, un cyclohexyle ou un benzyle, ou encore $R_{13}$ et $R_{14}$ forment ensemble, et avec l'atome d'azote auquel ils sont liés, un noyau hétérocyclique à 5 ou 6 maillons, $R_{15}$ représente l'hydrogène, un alkyle en $C_1$–$C_{12}$, un alcényle en $C_2$–$C_6$, un chlorométhyle, un cycloalkyle en $C_5$–$C_8$, un aralkyle en $C_7$–$C_{14}$, un phényle, un alkylphényle en $C_7$–$C_{10}$ ou un radical phényle, phénylméthyle ou phényléthyle porteur d'un ou de deux alkyles en $C_1$–$C_4$ et d'un hydroxy, $R_{16}$ représente l'hydrogène, un alkyle en $C_1$–$C_4$, un alcoxyalkyle en $C_3$ ou $C_4$, un phényle ou un phénoxyméthyle, $R_{17}$ représente l'hydrogène, un alkyle en $C_1$–$C_{12}$, un radical $-COR_{15}$, un radical $-CON(R_{13})(R_{14})$ ou un radical de formule:

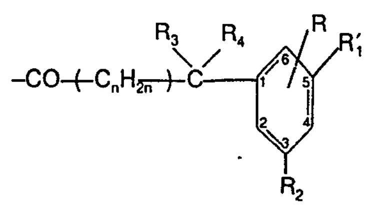

(dans laquelle $R'_1$ représente l'hydrogène ou un alkyle en $C_1$–$C_4$), et $R_{18}$ représente un alkyle en $C_1$–$C_{12}$, un phényle ou un alkylphényle en $C_7$–$C_{10}$, ou

$R_7$ représente un radical répondant à la formule:

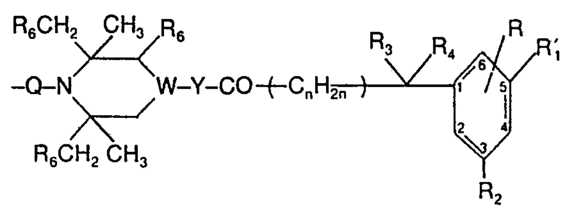

dans laquelle:

Q représente un radical $-(C_rH_{2r})-$ dont l'indice r peut prendre une valeur de 2 à 12, ou Q représente un alcénylène en $C_4$–$C_8$, un cycloalkylène en $C_5$–$C_{12}$, un phénylène, un xylylène, un bitolylène ou un radical $-CO-(C_rH_{2r})-CO-$,

Z représente –O– ou un radical $-N(R_{19})$ dans lequel $R_{19}$ représente l'hydrogène, un alkyle en $C_1$–$C_{18}$, un alcényle en $C_3$–$C_{12}$, un cycloalkyle en $C_3$–$C_{12}$, un phényle, un alkylaryle en $C_7$–$C_{14}$, un aralkyle en $C_7$–$C_{14}$, alcoxyalkyle en $C_2$–$C_{11}$ ou un

des radicaux $-COR_{20}$, $-COOR_{21}$, $-CON(R_{22})(R_{23})$, $-CH_2-CH(R_{24})-OR_{25}$, $-SOR_{26}$ et $-SO_2R_{26}$ dans lesquels $R_{20}$ a l'une des significations qui ont été données ci-dessus pour $R_{15}$ ou représente un noyau hétérocyclique, $R_{21}$ a l'une des significations qui ont été données pour $R_{12}$, $R_{22}$ l'une des significations qui ont été données pour $R_{13}$, $R_{23}$ l'une des significations qui ont été données pour $R_{14}$, $R_{24}$ l'une des significations qui ont été données pour $R_{16}$, $R_{25}$ l'une des significations qui ont été données pour $R_{17}$, et $R_{26}$ l'une des significations qui ont été données pour $R_{18}$, ou $R_{19}$ représente un radical de formule IV,

$R_{10}$ représente l'hydrogène, un alkyle en $C_1-C_{18}$, un phénoxyalkyle en $C_7-C_{23}$, un phényle, un aralkyle en $C_7-C_{14}$, un alcoxyalkyle en $C_2-C_{11}$ ou un radical de formule:

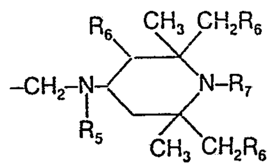 ,

$R_{11}$ représente l'hydrogène ou l'un des radicaux $-OR_{27}$, $-OCOR_{28}$, $-N(R_{29})-COR_{28}$, $-OSO_2R_{28}$ et $-N(R_{29})-SO_2R_{28}$ dans lesquels $R_{27}$ représente l'hydrogène, un alkyle en $C_1-C_{12}$, un allyle ou un benzyle, $R_{28}$ l'hydrogène, un alkyle en $C_1-C_{12}$, un alcényle en $C_2-C_6$, un chlorométhyle, un cycloalkyle en $C_5-C_8$, un phénylalkyle en $C_7-C_9$, un alkylphényle en $C_7-C_{10}$, un phényle ou un radical de formule:

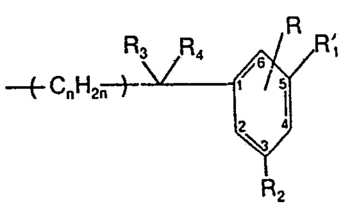

et $R_{29}$ représente l'hydrogène, un alkyle en $C_1-C_{12}$, un cycloalkyle en $C_5-C_8$ ou un benzyle, et

W représente l'un des radicaux suivants:

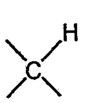

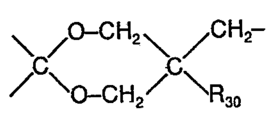

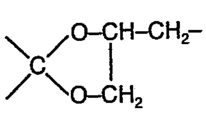

et

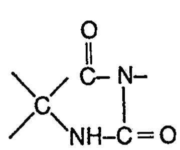

(où $R_{30}$ désigne un radical méthyle ou éthyle), avec la condition que, dans le cas où W représente un radical ayant une structure d'acétal cyclique, d et la somme (a+b+c) soient tous les deux nuls et que, dans le cas où W représente un radical avec une structure d'hydantoïne, d soit nul et la somme (a+b+c) différente de 0, les radicaux et symboles mentionnés à plusieurs reprises ayant toujours la signification donnée en premier lieu et, lorsqu'il y a plusieurs radicaux de formule:

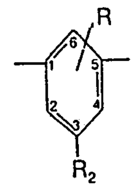

le radical R se trouvant toujours à la même position.

2. Matière pour enregistrement photographique en couleurs selon la revendication 1, matière caractérisée en ce que, dans les composés de formule I, le radical R se trouve à chaque fois à la position 6.

3. Matière pour enregistrement photographique en couleurs selon la revendication 1, matière caractérisée en ce que, dans les composés de formule I, le radical R se trouve à chaque fois à la position 4.

4. Matière pour enregistrement photographique en couleurs selon la revendication 1, matière caractérisée en ce que, dans les composés de formule I, le radical R se trouve à chaque fois à la position 2.

5. Matière pour enregistrement photographique en couleurs selon la revendication 1, matière caractérisée en ce qu'elle contient, comme stabilisant, au moins un composé polyalkyl-pipéridinique de formule Ia:

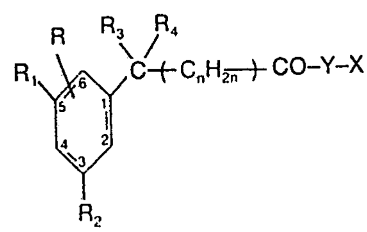

(Ia) .

6. Matière pour enregistrement photographique en couleurs selon la revendication 1, matière caractérisée en ce qu'elle contient, comme stabilisant, au moins un composé polyalkyl-pipéridinique répondant à la formule V:

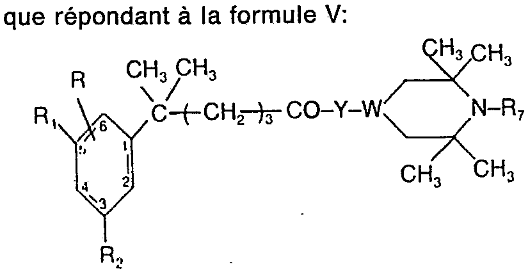

(V)

dans laquelle:

R représente un radical $-OH$ qui se trouve en position 2, 4 ou 6,

$R_1$ représente l'hydrogène, un alkyle en $C_1-C_4$, un radical de formule VI:

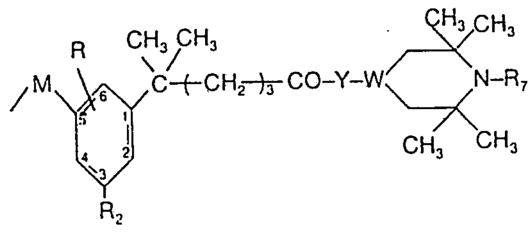

(VI)

ou un radical de formule VII:

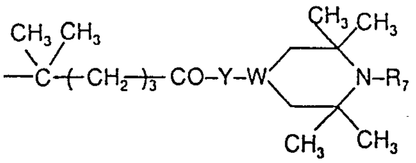

(VII) ,

$R_2$ représente un alkyle en $C_1$–$C_4$ ou un radical de formule VII,

Y représente –O–, ou un radical –N($R_5$)– dans lequel $R_5$ représente l'hydrogène, un alkyle en $C_1$–$C_{12}$, un cyclohexyle, un alcoxyalkyle en $C_3$ ou $C_4$ ou un radical de formule VIII:

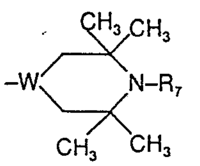

(VIII) ,

M représente un radical –CH($R_8$) ou –S–, le symbole $R_8$ désignant l'hydrogéne ou un alkyle en $C_1$–$C_4$,

$R_7$ représente un radical hydroxy, méthyle, allyle, benzyle, hydroxy-2 éthyle, acétyle ou acryloyle, ou un radical –CON($R_{13}$)($R_{14}$) dans lequel $R_{13}$ représente un alkyle en $C_1$–$C_8$, un cyclohexyle ou un phényle et $R_{14}$ l'hydrogène, un alkyle en $C_1$–$C_8$ ou un cyclohexyle, ou

$R_7$ représente un radical répondant à la formule:

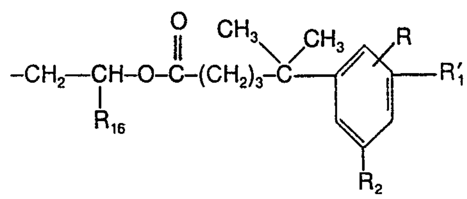

dans laquelle $R_{16}$ représente l'hydrogène ou un méthyle, et

W représente l'un des radicaux:

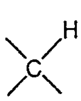

,

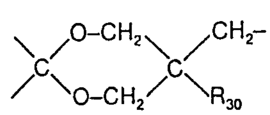

et

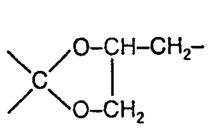

où $R_{30}$ représente un méthyle ou un éthyle, les symboles mentionnés à plusieurs reprises dans cette revendication ayant toujours la signification qui leur a été donnée la première fois dans cette revendication, et le substituant R du radical:

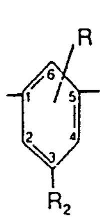

se trouvant toujours à la même position dans le cas où il y a plusieurs exemplaires de ce radical.

7. Matière pour enregistrement photographique en couleurs selon la revendication 6, caractérisée en ce que, dans les composés de formule V, le radical R se trouve à chaque fois à la position 6.

8. Matière pour enregistrement photographique en couleurs selon la revendication 6, caractérisée en ce que, dans les composés de formule V, le radical R se trouve à chaque fois à la position 4.

9. Matière pour enregistrement photographique en couleurs selon la revendication 6, caractérisée en ce que, dans les composés de formule V, le radical R se trouve à chaque fois à la position 2.

10. Matière pour enregistrement photographique en couleurs selon la revendication 1, matière caractérisée en ce qu'elle contient, comme stabilisant, un composé polyalkylpipéridinique répondant à la formule IX:

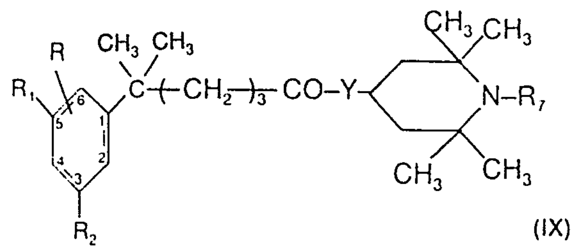

(IX)

dans laquelle:

R représente un radical –OH occupant l'une des positions 2, 4 et 6,

$R_1$ représente l'hydrogène, un alkyle en $C_1$–$C_4$ ou un radical de formule X:

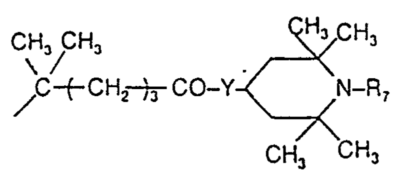

(X) ,

$R_2$ représente un alkyle en $C_1$–$C_4$ ou un radical de formule X,

Y représente –O– ou un radical –N($R_5$)– dans lequel $R_5$ représente l'hydrogène ou un alkyle en $C_1$–$C_8$, et

$R_7$ représente un radical hydroxy, méthyle, allyle, benzyle, hydroxy-2 éthyle, acétyle, acryloyle ou un radical répondant à la formule:

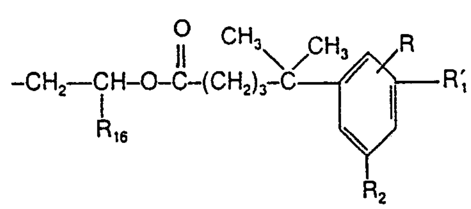

dans laquelle $R_{16}$ représente l'hydrogène ou un méthyle.

11. Matière pour enregistrement photographique en couleurs selon la revendication 10, caractérisée en ce que, dans les composés de formule IX, le radical R se trouve à chaque fois à la position 6.

12. Matière pour enregistrement photographique en couleurs selon la revendication 10, caractérisée en ce que, dans les composés de formule IX, le radical R se trouve à chaque fois à la position 4.

13. Matière pour enregistrement photographique en couleurs selon la revendication 10, caractérisée en ce que, dans les composés de formule IX, le radical R se trouve à chaque fois à la position 2.

14. Matière pour enregistrement photographique en couleurs selon la revendication 1, matière caractérisée en ce qu'elle contient, comme stabilisant, un composé polyalkylpipéridinique répondant à la formule XVI:

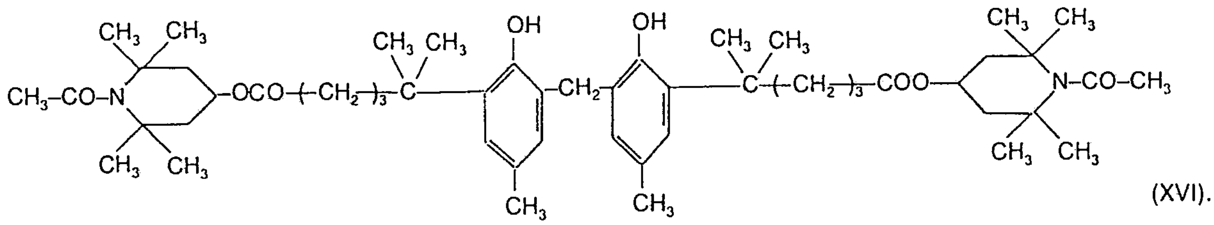

(XVI).

15. Matière pour enregistrement photographique en couleurs selon la revendication 1, matière caractérisée en ce qu'elle contient, comme stabilisant, un composé polyalkylpipéridinique répondant à la formule XVII:

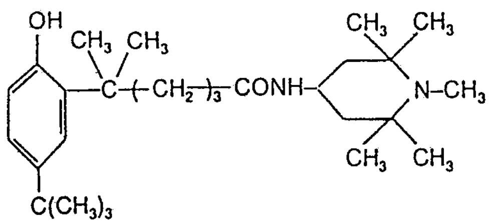

(XVII).

16. Matière pour enregistrement photographique en couleurs selon la revendication 1, matière caractérisée en ce qu'elle contient, comme stabilisant, un composé polyalkylpipéridinique répondant à la formule XVIII:

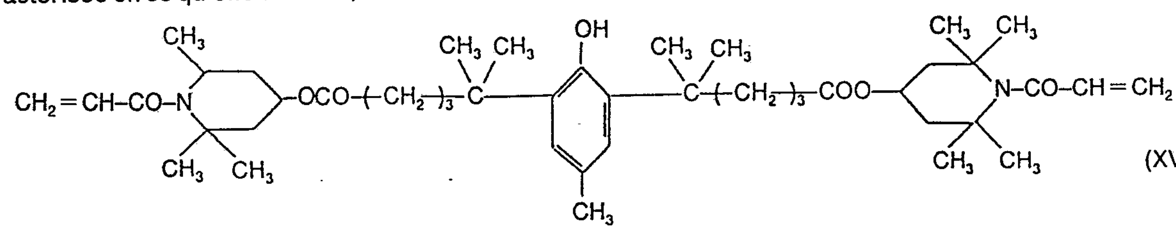

(XVIII).

17. Matière pour enregistrement photographique en couleurs selon la revendication 1, matière caractérisée en ce qu'elle contient, comme stabilisant, un composé polyalkylpipéridinique répondant à la formule XIX:

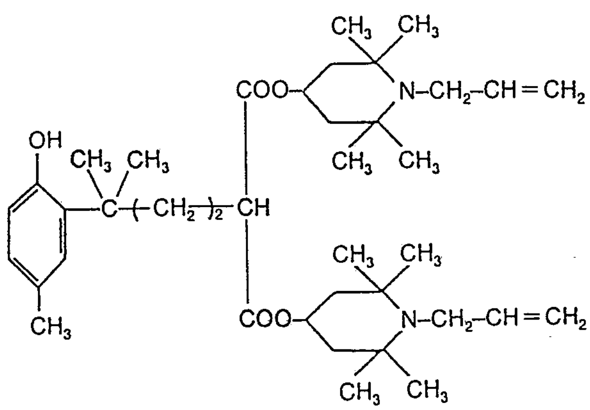

(XIX).

18. Matière pour enregistrement photographique en couleurs selon la revendication 1, matière caractérisée en ce qu'elle contient, comme stabilisant, un composé polyalkylpipéridinique répondant à la formule XX:

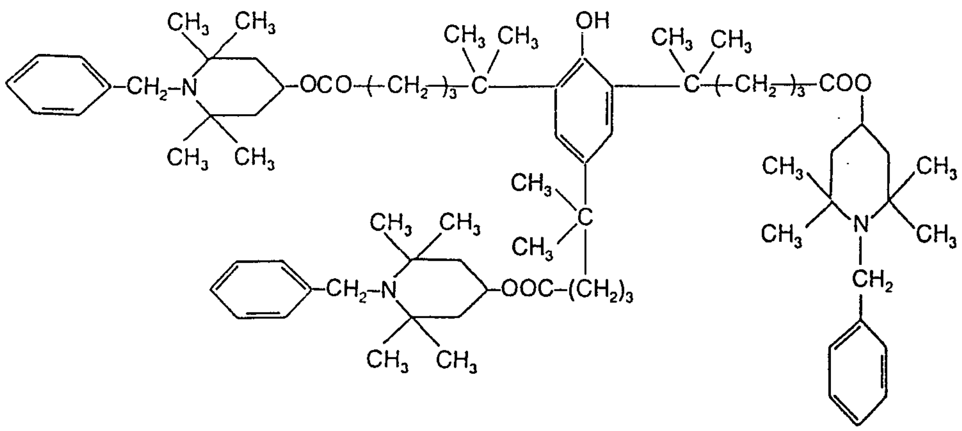

(XX).

19. Matière pour enregistrement photographique en couleurs selon la revendication 1, caractérisée en ce qu'elle contient les stabilisants de formule I en association avec des copulants bleu-vert, pourpres et jaunes.

20. Matière pour enregistrement photographique en couleurs selon la revendication 1, caractérisée en ce qu'elle contient les stabilisants de formule I en association avec des absorbeurs de rayons ultra-violets.

21. Matière pour enregistrement photographique en couleurs selon la revendication 20, caractérisée en ce que les absorbeurs de rayons ultra-violets sont des composés appartenant aux groupes des benzophénones, des acrylonitriles, des thiazolidones, des benzotriazoles, des oxazoles, des thiazoles ou des imidazoles.

22. Matière pour enregistrement photographique en couleurs selon la revendication 1, caractérisée en ce qu'elle contient, dans la même couche, les stabilisants de formule I en association avec des copulants bleu-vert, pourpres et jaunes et avec des absorbeurs de rayons ultra-violets.

23. Matière pour enregistrement photographique en couleurs selon la revendication 1, caractérisée en ce qu'elle contient de 1 à 2000 mg du stabilisant de formule I par mètre carré de la couche dans laquelle il est incorporé.

24. Procédé pour réaliser des images photographiques en couleurs, selon lequel on expose à la lumière, conformément à une image, une matière pour enregistrement photographique en couleurs selon la revendication 1 et on développe les couleurs.